# EUROPÄISCHE PATENTSCHRIFT

(11) **EP 1 325 299 B1**
(45) Veröffentlichungstag und Bekanntmachung des Hinweises auf die Patenterteilung: **15.12.2010**
(21) Anmeldenummer: 01978196.2
(22) Anmeldetag: 01.10.2001
(51) Int. Cl.: G01N 1/00

(54) **GAS- ODER HITZEMELDER, GASERZEUGER ODER HITZEERZEUGER, RAUCHGASERZEUGER SOWIE VERFAHREN ZUM PRÜFEN EINES GASMELDERS ODER EINES HITZEMELDERS UND VERFAHREN ZUM PRÜFEN EINES RAUCHGASMELDERS**
GAS OR HEAT DETECTOR, GAS OR HEAT GENERATOR, FLUE GAS GENERATOR, METHOD FOR TESTING A GAS DETECTOR OR A HEAT DETECTOR, AND METHOD FOR TESTING A FLUE GAS DETECTOR
DETECTEURS DE GAZ OU DE CHALEUR, GAZOGENE ET GENERATEUR DE CHALEUR ET DE GAZ DE FUMEES, PROCEDE POUR CONTROLER UN DETECTEUR DE GAZ OU DE CHALEUR ET PROCEDE POUR CONTROLER UN DETECTEUR DE GAZ DE FUMEES

(30) Priorität: 29.09.2000 DE 10048760; 30.01.2001 DE 10104330; 06.04.2001 DE 10117469; 09.05.2001 DE 10122572; 09.05.2001 US 289872 P; 10.05.2001 US 290133 P; 18.05.2001 US 291880 P; 15.08.2001 DE 10139033
(43) Veröffentlichungstag der Anmeldung: 09.07.2003
(60) Teilanmeldung: 08011642.9
(73) Patentinhaber: Tormaxx GmbH, 41199 Mönchengladbach (DE)
(72) Erfinder: KOCH, Hubert, 41199 Mönchengladbach (DE)
(74) Vertreter: Castell, Klaus
(86) Internationale Anmeldenummer: PCT/DE2001/003747
(87) Internationale Veröffentlichungsnummer: WO 2002/027293

(56) Entgegenhaltungen:
- CA-A- 2 083 990
- GB-A- 2 176 600

## Beschreibung

Die Erfindung betrifft einen Gasmelder oder einen Hitzemelder, einen Gaserzeuger oder einen Hitzeerzeuger, insbesondere für einen Gas- oder für einen Hitzemelder und einen Rauchgaserzeuger, vorzugsweise zum Simulieren eines realen Rauchgases sowie ein Verfahren zum Prüfen eines Gasmelders oder eines Hitzemelders und ein Verfahren zum Prüfen eines Rauchgasmelders, bei dem ein in der Nähe des Rauchgasmelders angeordneter Rauchgaserzeuger mittels eines Prüfmedium ein Rauchgas erzeugt und bei dem das Rauchgas eine Prüfung des Rauchgasmelders einleitet.

Gas- und Hitzemelder, insbesondere Rauchgasmelder dienen dazu, in Gebäuden durch spezielle Gase entstehende Luftverunreinigungen bzw. eine durch Feuer entstehende Hitze zu detektieren und ein Signal abzugeben. Dieses Signal kann ein optisches und/oder akustisches Signal sein. Vorzugsweise wird dieses Signal zur Auslösung eines Mechanismus genutzt, welcher der Rauchgasentstehung, der Rauchgas- oder der Feuerverbreitung entgegenwirkt. Hierzu sind beispielsweise Rolltore zu nennen, die sich bei derartigen Störfällen schließen, um beispielsweise eine Ausbreitung des Feuers zu verhindern.

Derartige Gasmelder, insbesondere Rauchgasmelder und/oder Hitzemelder, werden üblicherweise an einer Gebäudedecke angebracht, da sich die Rauchgase und/oder die Hitze in der Regel an der Gebäudedecke sammeln und hierbei eine schnelle Detektion des entstandenen Rauchgases und/oder Feuers erreicht werden kann. Gerade in größeren Hallen wie in Flughäfen oder Konzertsälen sind eine Vielzahl an Gas- und Hitzemeldern an der Decke angeordnet und über elektrische Leitungen mit einer Rauchgasmeldeanlage verbunden, die für die Auslösung der richtigen Mechanismen, wie beispielsweise das Schließen von Toren oder das Bedienen einer Sprinkleranlage, sorgen.

Derartige Anlagen haben einen hohen technischen Standard erreicht und sind in ihrer Funktion sehr zuverlässig. Trotzdem ist es vorgeschrieben, diese Anlagen in regelmäßigen Abständen zu überprüfen, damit unter anderem defekte Gasmelder ausgetauscht werden können. Hierzu werden die Gasmelder mit einem künstlich erzeugten Gas, insbesondere mit einem künstlich erzeugten Rauchgas in Verbindung gebracht, so dass sie einen Alarm auslösen, der direkt oder über eine Rauchgasmeldeanlage die vorgesehene Reaktion bewirkt. In Flughäfen kann beispielsweise durch das Anblasen eines Gasmelders mit Rauch das Schließen eines Rolltores ausgelöst werden.

Die Anordnung der Gasmelder im Deckenbereich führt vor allem dann zu Problemen, wenn extrem hohe Raumhöhen vorliegen und die Gasmelder dadurch schwer zugänglich sind. Auch bei geringeren Raumhöhen entsteht jedoch häufig das Problem, dass die Gasmelder hinter Verkleidungen angeordnet sind und daher schwer zugänglich sind.

Um insbesondere Rauchgasmelder hinsichtlich ihrer Funktionstüchtigkeit zu überprüfen, sind unter anderem mobile Rauchgaserzeuger bekannt. Diese Rauchgaserzeuger werden an einem Rauchgasmelder gehalten, bis dieser durch das künstlich erzeugte Rauchgas des Rauchgaserzeugers einen Alarm auslöst.

Hierbei gibt es Vorrichtungen, die ein Prüfaerosol in einer Druckflasche führen. Nachteilig bei diesen Vorrichtungen ist es, dass sie unter anderem wegen der Druckflasche unvorteilhaft zu handhaben sind. Auch ein Auslösen einer solchen Vorrichtung mittels eines meist komplizierten und aufwendigen Auslösemechanismuses ist unvorteilhaft durchzuführen.

Eine weitere Gattung von Prüfgeräten ist von der Firma Hekatron GmbH bekannt. Dieses Prüfgerät benutzt zur Rauchgasentwicklung ein Räucherstäbchen, welches in einem Gehäuse des Gerätes eingesetzt wird, wobei das Gehäuse mit großer Sorgfalt wieder verschlossen werden muss, da darauf zu achten ist, dass ein Dichtungsring, welcher einen Boden unter einem Oberteil des Gehäuses abdichtet, nicht beschädigt wird. Bei der eigentlichen Prüfung des Rauchgasmelders wird dann eine Schlauchspitze des Prüfgerätes an den Rauchgasmelder gehalten, wobei das Rauchgas durch Kompression eines Gummiballs aus der Schlauchspitze heraus an den Rauchgasmelder gerückt werden muss. Neben der vorhergehend beschriebenen umständlichen Handhabung muss bei einem Pumpzyklus ein Belüftungsloch des Gehäuses manuell mit einem Finger verschlossen werden, sodass aus diesem Belüftungsloch kein Rauchgas während der Kompression des Gummiballs entweichen kann. Um den zusammengedrückten Gummiball wieder mit Luft zu füllen, wird das Belüftungsloch des Gehäuses freigegeben, sodass über dieses Frischluft in den Gummiball des Prüfgerätes strömen kann. Ein solches Verfahren ist höchst umständlich, insbesondere dann, wenn die zu prüfenden Rauchgasmelder an der Decke hoch aufgehängt sind.

Bekannte Prüfgeräte für Gas- oder Hitzemelder haben wie beschrieben meist eine recht komplizierte Bedienung. Hinzu kommt noch, dass die Prüfprozedur mit den bekannten Prüfgeräten wegen häufiger Fehlversuche nicht selten mehrmals durchgeführt werden muss. Nachteilig ist es hierbei auch, dass durch einen längeren Verbleib der Prüfaerosole bzw. des Rauches des Räucherstäbchens in bzw. an dem Rauchgasmelder lange Wartezeiten entstehen, bis der Rauchgasmelder von einem Alarmzustand in den Normalzustand zurückgesetzt wird.

Der Erfindung liegt daher die Aufgabe zugrunde, Feuermeldeanlagen, insbesondere Gas- und Hitzemeldeanlagen, derart weiterzuentwickeln, dass diese leichter überprüft werden können.

Nach einem ersten Aspekt, stellt die vorliegende Erfindung ein System wie in Anspruch 1 beschrieben zur Verfügung. Entsprechend eines zweiten Aspekts, stellt die Erfindung, ein Prüfsystem gemäß Anspruch 16 zur Verfügung. Diese Aufgabe wird zum einen dadurch gelöst, dass ein Gasmelder oder ein Hitzemelder eine Prüfeinrichtung aufweist, welche mit dem Gasmelder oder mit dem Hitzemelder in Wirkverbindung steht.

Unter dem Begriff "Gas" versteht man hierbei alle gasförmigen Medien, wie beispielsweise auch Gase, die Feststoffpartikel aufweisen.

Hierzu zählen also alle Rauchgase, die Feststoffpartikel aufweisen, sodass hierdurch insbesondere auch Rauchgasmelder angesprochen werden können, die zum Detektieren eines Rauchgases geschaffen sind.

Besonders vorteilhaft ist es, wenn die Prüfeinrichtung einen Gaserzeuger und/oder einen Hitzeerzeuger umfasst. Hierdurch gelingt es der Prüfeinrichtung entsprechende Voraussetzungen zur Prüfung eines Gasmelders oder eines Hitzemelders zu erzeugen. Es versteht sich, das die Prüfeinrichtung für einen Gasmelder vorzugsweise einen Gaserzeuger aufweist und die Prüfeinrichtung für einen Hitzemelder vorzugsweise einen Hitzeerzeuger aufweist. Je nach Aufwendungsfall kann es sinnvoll sein, dass eine Prüfeinrichtung einen Gaserzeuger und einen Hitzeerzeuger umfasst.

Es versteht sich, dass die Erzeugung eines Rauchgases mit Feststoffpartikel auch durch eine Erzeugung eines Gases ohne Feststoffpartikel ersetzt werden kann. Dies ist insbesondere dann von Vorteil, wenn die Funktion eines Gasmelders überprüft werden soll, der auf das Vorhandensein eines von Feststoffpartikel freien Gases anspricht.

Es versteht sich des Weiteren, dass die Begriffe "Gasmelder" und "Gaserzeuger" ebenfalls alle technischen Einrichtungen implizieren, die ein Rauchgas detektieren bzw. erzeugen können. Dies bedeutet, dass der Begriff "Gasmelder" einen Rauchgasmelder und der Begriff "Gaserzeuger" einen Rauchgaserzeuger implizieren.

Der Erfindung liegt die Erkenntnis zugrunde, dass für wenigstens einen Gasmelder ein Gaserzeuger vorgesehen werden kann. Dieser Gaserzeuger kann dann in der Nähe des Gasmelders angeordnet werden. Durch das Auslösen des Gaserzeugers entsteht beispielsweise ein Rauchgas, das vom Gasmelder detektiert wird, so dass dieser sein Signal abgibt. Der Gaserzeuger kann beispielsweise stationär an Gebäudedecken angeordnet sein. Beispielsweise ist der Gaserzeuger durch eine individuelle Positionierung einem speziellen Gasmelder zugeordnet, sodass der Gaserzeuger in einer beliebigen Position gegenüber dem Gasmelder fixierbar ist. Es ist aber auch möglich, dass der Gaserzeuger und der Gasmelder eine körperliche Einheit bilden. Dies ist beispielsweise besonders vorteilhaft, wenn nur ein begrenzter Bauraum zur Anbringung eines Gasmelders und eines Gaserzeugers zur Verfügung steht oder wenn besondere Vorschriften hinsichtlich einer Bauaufsichtsbehörde eingehalten werden müssen.

Eine mögliche Ausführungsform des Systems aus einem Gasmelder und einem Gaserzeuger, insbesondere aus einem Rauchgasmelder und einem Rauchgaserzeuger, führt zwar dazu, dass gegenüber dem bekannten Stand der Technik eine Vielzahl an Rauchgaserzeugern notwendig wird. Da die Rauchgaserzeuger aber nicht mehr beim Prüfen der Anlage zum Gasmelder geführt werden müssen, sondern stationär eingebaut werden können, entsteht in der Regel ein hoher Kostenvorteil für den Betreiber der Anlage. Während bisher mit Leitern ein Rauchgaserzeuger zu einem speziellen Gasmelder gebracht werden musste oder Verkleidungen aufwendig entfernt werden mussten, genügt bei einer einmaligen Installation der Rauchgaserzeuger jeweils in der Nähe eines Gasmelders eine Auslösung des Rauchgaserzeugers, um den zugehörigen Gasmelder zu prüfen.

Nur wenn das Zusammenspiel von Gaserzeuger und Gasmelder, insbesondere von Rauchgaserzeuger und Rauchgasmelder, nicht funktionieren sollte, muss der Techniker die Anlage untersuchen. Sofern das Zusammenspiel jedoch funktioniert, ist sichergestellt, dass der Gasmelder in Ordnung ist und es erübrigen sich somit vorteilhafter Weise weitere Untersuchungen.

Da bei den regelmäßigen Untersuchungen der Gasmelder in der Regel festgestellt wird, dass die Gasmelder in Ordnung sind, und nur im Ausnahmefall einzelne Gasmelder ausgetauscht werden müssen, erleichtert der erfindungsgemäße Gasmelder die Arbeit des Serviceunternehmens und führt somit zu einem beträchtlichen Kostenvorteil für den Anlagenbetreiber.

Eine einfache Nachrüstung von Anlagen mit dem erfindungsgemäßen Gasmelder wird dadurch erzielt, dass neben den bekannten bisher eingesetzten Gasmeldern ein Gaserzeuger angeordnet wird. Dadurch entsteht ein erfindungsgemäßer Gasmelder bei dem der Gaserzeuger vorzugsweise unmittelbar neben dem Gasmelder angeordnet ist. Der Gaserzeuger kann als einfaches Bauteil bei bestehenden mit Gasmeldern ausgerüsteten Gebäuden beispielsweise an der Decke neben dem Gasmelder angeschraubt sein. Dies führt zu einer kostengünstigen Nachrüstung bestehender Anlagen und somit sofort ohne hohe Investitionen zu einer Reduktion des Wartungsaufwandes.

Insbesondere für Neuanlagen wird vorgeschlagen, dass der Gaserzeuger in den Gasmelder integriert ist. Dadurch wird eine einfache kompakte Anlage bereitgestellt, die sowohl das Gasüberwachungssystem als auch das eigene Kontrollsystem bereitstellt. Die Integration von Gaserzeuger und Gasmelder führt weiterhin zu dem Vorteil, dass Anlagenteile des Gasmelders wie beispielsweise die Stromversorgung auch für den Gaserzeuger genutzt werden können.

Da Gasmeldeanlagen, insbesondere Rauchgasmeldeanlagen, in regelmäßigen Abständen überprüft werden müssen, wird vorgeschlagen, dass der Gasmelder eine Zeituhr aufweist. Diese Zeituhr erlaubt es, vorprogrammiert in regelmäßigen zeitlichen Abständen den Gasmelder zu prüfen, indem zu vorgegebenen Zeiten mittels des Gaserzeugers beispielsweise ein Rauchgas abgegeben wird, welches zu der vorbestimmten Reaktion am Gasmelder führen muss. Sofern die vorgesehene Reaktion nicht bewirkt wird, ist eine genauere Untersuchung des Gasmelders und gegebenenfalls des Gaserzeugers notwendig.

Eine vorteilhafte Ausführungsvariante sieht vor, dass der Gaserzeuger eine Fernsteuerung aufweist. Eine derartige Fernsteuerung kann mittels einer Leitung oder kabellos ausgeführt sein und erlaubt es, zu beliebigen Zeiten den Gaserzeuger auszulösen. Dies dient in der Regel dazu, den Gasmelder zu prüfen. Die Fernsteuerung kann jedoch auch dazu dienen, die durch den Gasmelder ausgelöste Reaktion zu bewirken. Beispielsweise kann über diese Fernsteuerung ein Rolltor geschlossen werden, indem beispielsweise ein Rauchgas erzeugt wird, das auf den Gasmelder wirkt und somit das Schließen des Rolltores auslöst.

Vorteilhaft ist es, wenn der Gaserzeuger elektrisch auslösbar ist. Der Gaserzeuger kann hierzu mit einer Stromversorgung versehen sein, an die nur dann Strom angelegt wird, wenn beispielsweise Rauchgas erzeugt werden soll.

Eine Ausführungsvariante sieht vor, dass der Gaserzeuger als stromunabhängiges Bauteil ausgebildet ist. Die für die Rauchgaserzeugung oder Rauchgasfreigabe notwendige Energie wird hierbei durch eine vorzugsweise wiederaufladbare Batterie bereitgestellt. Derartige Gaserzeuger sind insbesondere als drahtlos ferngesteuerte Elemente einfach einbaubar und leicht bedienbar.

Dem Fachmann ist bekannt, dass es verschiedene Möglichkeiten gibt, die je nach Anwendungsfall notwendigen Rauchgase zu erzeugen.

Eine einfache Ausführungsvariante sieht vor, dass der Gaserzeuger eine Gaspatrone aufweist. Diese Gaspatrone ist mit einem unter Überdruck stehendem Rauchgas gefüllt, so dass bei einem Öffnen der Gaspatrone solange Rauchgas freigesetzt wird, wie die Gaspatrone geöffnet gehalten wird. Die Gaspatrone kann mit verschiedenen Gasarten oder einer Gasmischung gefüllt sein und es können auch verschiedene Gaspatronen im Gasmelder angeordnet sein, um das Ansprechen des Gasmelders auf verschiedene Gasarten, insbesondere auf ein Rauchgas, zu prüfen.

Eine andere Ausführungsvariante sieht vor, dass der Gaserzeuger einen Fluidbehälter und eine Heizeinrichtung aufweist. Die Heizeinrichtung sorgt hierbei dafür, dass bei Auslösen des Gaserzeugers Flüssigkeit verdampft und somit beispielsweise ein Rauchgas freigegeben wird. Hierbei ist es von Vorteil, wenn die Heizeinrichtung nur dann erhitzt wird, wenn das Rauchgas erzeugt werden soll. Der Flüssigkeitsbehälter kann auch als Überdruckbehälter so ausgebildet sein, dass er es erlaubt, die Flüssigkeit zu versprühen. Diese Flüssigkeit kann in Zusammenwirkung mit der Heizeinrichtung durch Verdunstung oder Verbrennen ein Gas erzeugen, oder die Flüssigkeit bzw. ein aus dem Fluidbehälter austretendes Gas wird entzündet, so dass die Heizeinrichtung direkt durch Verbrennen des austretenden Fluids ein entsprechendes Gas oder eine Hitze erzeugt.

Um das entstehende Gas gezielt dem Gasdetektor zuzuführen, wird vorgeschlagen, dass der Gaserzeuger ein Gebläse aufweist. Dieses Gebläse muss nur dann angeschaltet werden, wenn beispielsweise Rauchgas erzeugt wird. Außerdem ist es möglich, den mit beispielsweise Rauchgas temporär kontaminierten Gasmelder nach dem Detektieren des Rauchgases wieder frei zu blasen. Hierdurch verkürzt sich ein Prüfzyklus wesentlich, da der Gasmelder wieder schneller von einem Alarmzustand auf einen Normalzustand zurück schaltet.

Da es beispielsweise hinsichtlich Installations- oder Wartungsarbeiten schwierig sein kann, unter Druck stehende Patronen oder flüssigkeitsgefüllte Behälter zu transportieren, wird vorgeschlagen, dass der Gaserzeuger einen Festkörper aufweist, der bei Erhitzung zumindest zum Teil verdampft. Dieser Festkörper kann ein Kunststoffelement oder ein Wachs sein. Dieses Wachs wird vorzugsweise durch ein sich bei Stromdurchfluss erwärmendes Widerstandselement erhitzt, so dass bei Stromdurchfluss durch die Erwärmung ein Teil des Festkörpers verdampft. Ein derartiger wachsartiger Festkörper kann ein wasserklares Gel ohne Geruch sein. Gut geeignet sind Gele aus Kohlewasserstoffen im Bereich der Weißöle, die unter Zusatz eines Gelbildners hergestellt sind. Derartige Gele haben vorzugsweise einen Siedepunkt, der über 250 °C liegt. Der Schmelzpunkt liegt vorzugsweise bei etwa 80 °C. Dies führt dazu, dass bei derartigen Stoffen unter Raumtemperatur praktisch kein Verdampfen von Bestandteilen stattfindet. Bei der Lagerung der Stoffe oder aus dem im Prüfgerät befindlichen Vorrat kann daher eine Belastung der Umgebungsluft sicher ausgeschlossen werden.

In der Praxis werden langkettige aliphatische Kohlenwasserstoffe verwendet, von denen je Prüfvorgang etwa 1mg verdampft wird. Diese verdampfte Menge ist ohne gesundheitliche Relevanz, da aliphatische langkettige Kohlenwasserstoffe nur in hohen Konzentrationen zur mechanischen Reizung der oberen Atemwege führen. Die beschriebenen Stoffe haben darüber hinaus den Vorteil, dass sie sich in der Nähe der Stoffabgabe absetzen und die freigesetzten Mengen an Material weder zur Korrosion noch anderen negativen Einflüssen auf benachbarten elektronischen oder mechanischen Bauteilen führen.

Diese Substanz kann insbesondere bei allen in dieser Anmeldung beschriebenen Rauchgaserzeugern vorteilhaft eingesetzt werden.

Die der Erfindung zu Grunde liegende Aufgabe wird zum anderem dadurch gelöst, dass ein Hitzemelder, insbesondere ein Feuermelder, einen Hitzeerzeuger aufweist, mit dem er in Wirkverbindung steht.

Unter dem Begriff "Hitzeerzeuger" sind sämtliche technischen Vorrichtungen zu verstehen, mit denen sich eine Hitze erzeugen lässt, welche ausreichend heiß wird, um von einem Hitzemelder detektiert zu werden. Es versteht sich, dass als Hitzeerzeuger elektrische Vorrichtungen, Vorrichtungen mit einer offenen Flamme oder ähnliche Vorrichtungen zur Erzeugung einer Hitze herangezogen werden können.

Ähnlich wie vorhergehend schon bei dem Gasmelder und dem Gaserzeuger beschrieben, ist es möglich, dass auch für wenigstens einen Hitzemelder ein Hitzeerzeuger vorgesehen werden kann. Dieser Hitzeerzeuger kann dann ebenfalls in der Nähe des Hitzemelders angeordnet werden. Der Hitzeerzeuger produziert hierbei eine derart große Wärme, dass diese von dem Hitzemelder detektiert wird, sodass dieser ein Signal abgibt. Der Hitzeerzeuger kann hierbei beispielsweise stationär an Gebäudedecken angeordnet sein. Durch seine jeweilige frei wählbare Positionierung ist er einem speziellen Hitzemelder zugeordnet.

Das erfindungsgemäße System aus einem Hitzemelder, insbesondere einen Feuermelder, und einen Hitzeerzeuger führt zwar dazu, dass gegenüber dem bekannten Stand der Technik eine Vielzahl an Hitzeerzeugem notwendig wird. Da die Hitzeerzeuger aber nicht mehr beim Prüfen der Anlage zum Hitzemelder gebracht werden müssen, sondern stationär eingebaut werden können, entsteht in der Regel ein hoher Kostenvorteil für den Betreiber der Anlage. Während bisher beispielsweise mit Leitern ein Hitzeerzeuger zu einem speziellen Hitzemelder hingeführt werden oder Verkleidungen aufwendig entfernt werden mussten, genügt bei einer einmaligen Installation der Hitzeerzeuger jeweils in der Nähe eines Hitzemelders eine Auslösung des Hitzeerzeugers, um den zugehörigen Hitzemelder zu prüfen.

Nur wenn das Zusammenspiel von Hitzeerzeuger und Hitzemelder, insbesondere Feuermelder, nicht funktionieren sollte, muss der Techniker die Anlage untersuchen und gegebenenfalls instandsetzen. Sofern das Zusammenspiel jedoch funktioniert, ist sichergestellt, dass der Hitzemelder in Ordnung ist und es erübrigen sich somit weitere Untersuchungen.

Da bei den regelmäßigen Untersuchungen der Hitzemelder in der Regel festgestellt wird, dass die Hitzemelder in Ordnung sind, und nur in Ausnahmefall einzelne Hitzemelder ausgetauscht werden müssen, erleichtert der erfindungsgemäße Hitzemelder die Arbeit des Serviceunternehmens und führt somit zu einem beträchtlichen Kostenvorteil für den Anlagenbetreiber.

Eine einfache Nachrüstung von Anlagen mit dem erfindungsgemäßen Hitzemelder wird dadurch erzielt, dass neben den bekannten bisher eingesetzten Hitzemeldern ein Hitzeerzeuger angeordnet ist. Dadurch entsteht ein erfindungsgemäßer Hitzemelder, insbesondere ein Feuermelder, bei dem der Hitzeerzeuger neben dem Hitzemelder angeordnet ist. Der Hitzeerzeuger kann als einfaches Bauteil bei bestehenden mit Hitzemeldern ausgerüsteten Gebäuden beispielsweise an der Decke neben dem Hitzemelder angeschraubt sein. Dies führt zu einer kostengünstigen Nachrüstung bestehender Anlagen und somit sofort ohne hohe Investition zu einer Reduktion des Wartungsaufwandes. Insbesondere für Neuanlagen wird vorgeschlagen, dass der Hitzeerzeuger in dem Hitzemelder, insbesondere in dem Feuermelder, integriert ist. Dadurch wird eine einfache kompakte Anlage bereitgestellt, die sowohl das Feuerüberwachungssystem als auch das eigene Kontrollsystem bereitstellt. Die Integration von Hitzeerzeuger und Hitzemelder führt weiterhin zu dem Vorteil, dass Anlagenteile des Hitzemelders wie beispielsweise eine Stromversorgung auch für den Hitzeerzeuger benutzt werden können.

Da Hitzemeldeanlagen in regelmäßigen Abständen überprüft werden müssen, wird vorgeschlagen, dass der Hitzemelder eine Zeituhr aufweist. Diese Zeituhr erlaubt es, vorprogrammiert in regelmäßigen zeitlichen Abständen den Hitzemelder zu prüfen, in dem zu vorgegebenen Zeiten mittels des Hitzeerzeugers derart Hitze erzeugt wird, so dass dies zu einer vorbestimmten Reaktion am Hitzemelder führen muss. Sofern die vorgesehene Reaktion nicht bewirkt wird, ist eine genauere Untersuchung des Hitzemelders und gegebenenfalls des Hitzeerzeugers notwendig.

Eine vorteilhafte Ausführungsform sieht vor, dass der Hitzeerzeuger eine Fernsteuerung aufweist. Eine derartige Fernsteuerung kann mittels einer Leitung oder kabellos ausgeführt sein und erlaubt es, zu beliebigen Zeiten den Hitzeerzeuger auszulösen. Dies dient in der Regel dazu, den Hitzemelder zu prüfen. Die Fernsteuerung kann jedoch auch dazu dienen, die durch den Hitzemelder ausgelöste Reaktion zu bewirken. Beispielsweise kann über diese Fernsteuerung ein Rolltor geschlossen werden, in dem mittels des Hitzeerzeugers eine Hitze erzeugt wird, die auf den Hitzemelder wirkt und somit das Schließen des Rolltores auslöst. Vorteilhaft ist es, wenn der Hitzeerzeuger elektrisch auslösbar ist. Der Hitzeerzeuger kann hierzu mit einer Stromversorgung versehen sein, an die nur dann Strom angelegt wird, wenn Hitze erzeugt werden soll.

Dem Fachmann ist bekannt, dass es verschiedene Möglichkeiten gibt, die je nach Anwendungsfall notwendige Hitze zu erzeugen. Ein einfaches Ausführungsbeispiel sieht vor, dass der Hitzeerzeuger eine elektrische Heizeinrichtung aufweist. Diese Heizeinrichtung ist beispielsweise in der Nähe eines entsprechenden wärmeempfindlichen Sensors des Hitzemelders angeordnet. Durchfließt nun ein Strom einen metallischen Draht der elektrischen Heizeinrichtung, so erwärmt sich der Draht derart, dass der Sensor des Hitzemelders diese Wärmequelle detektiert.

Eine andere Ausführungsvariante sieht vor, dass der Hitzeerzeuger ein Gebläse aufweist. Beispielsweise ist eine Heizeinrichtung nicht unmittelbar an einem Sensor des Hitzemelders angeordnet, sondern beabstandet von ihm. Um die Hitze nun effektiv in die Nähe des Hitzemelders zu bringen, wird das Gebläse eingeschaltet, wenn die Heizeinrichtung des Hitzeerzeugers aktiv ist. Hierbei gelangt etwa heiße Luft an den Sensor des Hitzemelders.

Eine Ausführungsvariante sieht vor, dass der Hitzeerzeuger als stromunabhängiges Bauteil ausgebildet ist. Die für die Hitzeerzeugung notwendige Energie wird hierbei durch eine vorzugsweise wiederaufladbare Batterie bereitgestellt. Derartige Hitzeerzeuger sind insbesondere als drahtlos ferngesteuerte Elemente einfach einbaubar und leicht bedienbar.

Eine weitere Ausführungsvariante sieht vor, dass der Hitzeerzeuger einen Fluidbehälter und eine Zündeinrichtung aufweist. Der Fluidbehälter beinhaltet hierbei ein brennbares Medium, welches beispielsweise durch eine Düse in die Umgebung strömen kann und hierbei von einer Zündeinrichtung entzündet wird, so dass hierbei eine Flamme entsteht. Beispielsweise ist der Hitzeerzeuger dann derart angeordnet, dass die Flamme unmittelbar unter dem Hitzemelder angeordnet ist, so dass durch die aufsteigende Hitze der Hitzemelder ausgelöst wird. Es ist ebenfalls möglich, dass die Flamme eine Metalleinrichtung erhitzt, an der ein Sensor des Hitzemelders angeordnet ist, so dass bei Erhitzung des Metalles der Hitzemelder aktiviert wird. Ist beispielsweise eine Anordnung eines mit Fluid betriebenen Hitzeerzeugers nicht unmittelbar in der Nähe des Hitzemelders möglich, kann auch hier ein Gebläse die Hitze der Flamme in die Nähe des Hitzemelders bringen.

Das Medium des Fluidbehälters kann hierbei unter Druck stehen oder auch drucklos sein.

Eine weitere Ausführungsvariante sieht vor, dass der Hitzeerzeuger einen Festkörper aufweist, der bei Aktivierung eine Wärmestrahlung abgibt. Beispielsweise ist dies eine Infrarotlampe, die in unmittelbarer Nähe des Hitzemelders angeordnet ist, so dass bei deren Aktivierung die Wärmestrahlung der Infrarotlampe ausreicht, den Hitzemelder zu aktivieren.

Die der Erfindung zugrundeliegende Aufgabe wird auch durch einen Gaserzeuger oder Hitzeerzeuger, insbesondere für einen Gas- oder für einen Hitzemelder gelöst, der einen Fluidbehälter und ein Kapillarrohr aufweist, wobei ein Ende des Kapillarrohres im Fluidbehälter angeordnet ist und das andere Ende eine Heizeinrichtung aufweist und wobei die Heizeinrichtung eine Fernsteuerung aufweist.

Ein derartiger Gaserzeuger ist besonders einfach im Aufbau und lässt sich entweder in bekannte Gasmelder integrieren oder neben bekannten Gasmeldern anordnen. Das Kapillarrohr fördert einen kleinen Strom eines Fluids zu einer Heizeinrichtung, die - sofern sie angeschaltet ist - für eine Verdunstung oder ein Verdampfen des Fluids sorgt. Um nur zu vorgegebenen Zeiten beispielsweise eine Rauchgaserzeugung zu bewirken, weist die Heizeinrichtung eine Fernsteuerung auf, mittels derer sie angestellt wird. Dieser einfache Aufbau eines Gaserzeugers zeigt, dass mit einfachen Mitteln der hohe Serviceaufwand insbesondere bei Rauchgasmeldeanlagen reduziert werden kann, ohne dass hierunter die Sicherheit leidet.

Um ein Überhitzen des Prüfmediums zu vermeiden, besteht die Möglichkeit, die Heizdauer der Heizeinrichtung temporär zu begrenzen, sodass für ein erneutes Aktivieren der Heizeinrichtung ein Schalter entsprechend betätigt werden muss.

Eine bevorzugte Ausführungsform sieht vor, dass der Gaserzeuger einen Wärmeleitkörper aufweist. Beispielsweise ist der Wärmeleitkörper aus einem elektrisch leitenden Metallblech hergestellt, so dass sich die beheizbare Oberfläche erhöht. Hierbei ist die Oberfläche des Metallblechs weit aus größer als die Oberfläche des Drahtes.

Besonders vorteilhaft ist es, wenn der Wärmeleitkörper einen elektrischen Widerstand aufweist. Beispielsweise ist dieser Widerstand ein handelsüblicher ohmscher Widerstand, so dass hierdurch ein Produkt aus der Massenfertigung zur Leistungssteigerung der Heizeinrichtung herangezogen werden kann.

Gemäß einer weiteren Ausführungsform, ist es vorteilhaft, wenn der Wärmeleitkörper ein poröses Material aufweist. Die Porosität des Materials erlaubt es, eine weitere Substanz nach Art eines Schwammes aufzusaugen, so dass die Substanz mit dem Wärmeleitkörper im Bereich der Poren in einem innigen Kontakt steht. Wird der Wärmeleitkörper bzw. der elektrische Widerstand erhitzt, verdampft die in dem porösen Material eingelagerte Substanz, wodurch beispielsweise ein Rauchgas entsteht.

Hat der Wärmeleitkörper selbst keinen porösen Körper, ist es von Vorteil, wenn an dem Wärmeleitkörper ein poröses Bauteil angeordnet ist. Auch in diesem porösen Bauteil kann eine Substanz in dessen Poren eingelagert werden.

Um beispielsweise eine Rauchgaserzeugung an einer gezielten Stelle des porösen Bauteils zu erzielen und/oder um ein unkontrolliertes Austreten der Substanz aus dem porösen Bauteil zu verhindern, ist es vorteilhaft, wenn der poröse Körper oder das poröse Bauteil eine Ummantelung aufweist, vorzugsweise eine hitzebeständige Folie. Durch die hitzebeständige Folie wird die durch das poröse Bauteil erhitzte Substanz daran gehindert, sich an einer unerwünschten Stelle des porösen Bauteils zu verflüchtigen.

Vorteilhafter Weise hat die Ummantelung wenigstens eine Öffnung. Beispielsweise weist die hitzebeständige Folie eine Öffnung auf, durch welche die erhitzte Substanz verdampft bzw. verraucht.

Eine besonders bevorzugte Ausführungsform sieht vor, dass der Gaserzeuger eine Schnittstelle zu einem Netzwerk aufweist. Beispielsweise ist der Gaserzeuger an ein lokales Netzwerk eines Gebäudes angeschlossen, so dass der Gaserzeuger beispielsweise von einer zentralen Einrichtung aus angesteuert werden kann. Die Schnittstelle kann hierbei sowohl kabelgebunden als auch kabellos ausgebildet sein. Es ist ebenfalls möglich, dass der Gaserzeuger nicht nur zu einem lokalen Netzwerk einen Kontakt aufweist, sondern vielmehr auch zu einem weiträumigen Netzwerk. Beispielsweise ist ein Gaserzeuger über ein weiträumiges Netzwerk mit einem zentralen Sicherheitsdienst verbunden, welcher sich nicht unmittelbar in dem Gebäude der zu prüfenden Gasmelder befindet.

Es versteht sich, dass der vorhin beschriebene Hitzeerzeuger ebenfalls mittels einer Schnittstelle mit einem Netzwerk verbunden sein kann. Hierbei ergeben sich die selben Vorteile wie bei dem Gaserzeuger.

Die der Erfindung zugrunde liegende Aufgabe wird auch gelöst von einem Rauchgaserzeuger zum Simulieren eines realen Rauchgases, wobei der Rauchgaserzeuger zur Erzeugung des Rauchgases eine elektrische Heizeinrichtung aufweist. Eine solche elektrische Heizeinrichtung kann in vielfältiger Form vorliegen, besonders bevorzugt wird jedoch ein Wärmeerzeuger, der einen elektrisch leitfähigen Draht aufweist. Vorteilhaft bei diesem Wärmeerzeuger ist es, dass hierbei eine Hitze erzeugt werden kann, ohne dabei eine offene Flamme zu verwenden. Zur Hitzeerzeugung wird lediglich ein Strom durch den Draht geleitet, sodass dieser sich erhitzt. Somit ist insbesondere eine elektrische Heizeinrichtung besonders vorteilhaft.

Eine bevorzugte Ausführungsvariante sieht vor, dass die Heizeinrichtung ein elektrischer Widerstand ist. Vorzugsweise ist dies ein handelsüblicher ohmscher Widerstand, der auf einfache Weise in einen elektrisch leitfähigen Draht eingefügt werden kann. Insbesondere der ohmsche Widerstand verstärkt zum einen die Hitzeerzeugung und zum anderen erhöht er die sich erhitzende Oberfläche, welche im Stande ist eine Hitze abzugeben.

Besonders vorteilhaft ist es, wenn der Rauchgaserzeuger ein elektrisches Gebläse aufweist. Das elektrische Gebläse kann hierbei ein kleiner Ventilator sein, der aus der Umgebung Luft ansaugt und diese dann durch den Rauchgaserzeuger leitet, wobei die Luft das Rauchgas durch wenigstens eine Öffnung des Rauchgaserzeugers in Richtung eines Rauchgasmelders führt. Es ist möglich, anstatt des Ventilators jede andere Einrichtung zu verwenden, die in der Lage ist Luft zu beschleunigen.

Eine weitere Ausführungsvariante sieht vor, dass der Rauchgaserzeuger eine elektrische Energiequelle aufweist. Mittels dieser elektrischen Energiequelle ist beispielsweise eine Stromversorgung der Heizeinrichtung und des Gebläses gewährleistet. Als elektrische Energiequelle kann hierbei ein öffentliches Stromnetz oder eine Solarstromeinheit dienen. Bevorzugt besitzt die elektrische Energiequelle jedoch eine Batterie oder einen wiederaufladbaren Akkumulator.

Besonders vorteilhaft ist es, wenn der Rauchgaserzeuger elektrisch auslösbar ist. Hierbei wird lediglich dann ein Rauchgas erzeugt, wenn die Heizeinrichtung bzw. der Wärmeerzeuger elektrisch ausgelöst wird, wobei dann durch die Heizeinrichtung bzw. durch den Wärmeerzeuger ein Strom der elektrischen Energiequelle fließt.

Nach der Erfindung ist vorgeschlagen, dass der Rauchgaserzeuger einen Wärmeleitkörper aufweist. Beispielsweise ist der Wärmeleitkörper aus einem elektrisch leitenden Metallblech hergestellt, sodass sich durch die gegenüber der Oberfläche des Drahtes relativ große Oberfläche des Metallbleches die heizbare Oberfläche erhöht. Besonders vorteilhaft ist es, wenn der Wärmeleitkörper in Gestalt eines elektrischen Widerstands realisiert ist. Beispielsweise ist dieser Widerstand ein handelsüblicher Widerstand, sodass hierdurch ein Produkt aus der Massenfertigung zur Leistungssteigerung der Heizeinrichtung herangezogen werden kann.

Gemäß einer weiteren Ausführungsvariante ist vorgesehen, dass der Rauchgaserzeuger eine Wärmekammer aufweist, in der ein Prüfmedium angeordnet ist. Vorteilhafterweise wird mittels des Prüfmediums das Rauchgas erzeugt.

Bevorzugt ist die Heizeinrichtung in der Wärmekammer angeordnet, so dass sie sich in der unmittelbaren Umgebung des Prüfmediums befindet.

Das Prüfmedium kann fest oder flüssig sein, besonders vorteilhaft ist es, wenn das Prüfmedium ein gelartiges Material umfasst, welches bei der Erhitzung zumindest teilweise verraucht. Beispielsweise befindet sich in dem gelartigen Prüfmedium die elektrische Heizeinrichtung bzw. der Wärmeleitkörper, insbesondere der handelsübliche Widerstand, sodass bei der Erhitzung ein Teil des Prüfmediums auf Grund der unmittelbaren Nähe der Heizeinrichtung so stark erhitzt wird, dass es in einen gasförmigen Zustand verdampft und der Rauchgaserzeuger ein Rauchgas erzeugt.

Es wurde gefunden, dass es vorteilhaft ist, wenn das Prüfmedium lediglich eine Masse von weniger als 5 g, vorzugsweise von weniger als 1 g, aufweist. Vorteilhafterweise benötigt der erfindungsgemäße Rauchgaserzeuger für eine Prüfsequenz lediglich ca. 0,001 g des gelartigen Prüfmediums, sodass mit dem erfindungsgemäßen Rauchgaserzeuger bis zu 600 Prüfungen durchgeführt werden, ohne den Rauchgaserzeuger mit einem Prüfmedium neu zu befüllen. Dies entspricht bei einem monatlichen Prüfzyklus einer Einsatzdauer von ca. 40 Jahren. Vorteilhafterweise lässt sich der Rauchgaserzeuger mit einem neuen Prüfmedium nachfüllen. Durch die geringe Menge an Prüfmedium verringert sich das Gewicht des Rauchgaserzeugers erheblich.

Nach einer weiteren Ausgestaltung der Erfindung ist vorgesehen, dass die Heizeinrichtung mit dem Prüfmedium in Wirkkontakt steht. Beispielsweise ist die Heizeinrichtung in der Wärmekammer derart angeordnet, dass sie unmittelbar von dem Prüfmedium umschlossen ist. Hierdurch ist der Aufbau eines Rauchgaserzeugers sehr einfach konstruiert, wobei zusätzliche Mittel zur Rauchgasentwicklung überflüssig sind.

Letztlich ist vorgeschlagen, dass der Rauchgaserzeuger eine Kapillareinrichtung aufweist. Beispielsweise ist ein Ende eines Kapillarrohres in einem Gehäuse, in welchem sich das Prüfmedium befindet, angeordnet. Das andere Ende des Kapillarrohres weist dagegen eine Heizeinrichtung auf oder steht mit der Heizeinrichtung unmittelbar in Wirkverbindung. Durch das Kapillarrohr bewegt sich mittels Adhäsionskräften immer ein Teil des Prüfmediums in den Bereich der Heizeinrichtung, die bei einer Aktivierung für ein Verrauchen des Prüfmediums sorgt.

Vorzugsweise ist die Heizeinrichtung fernsteuerbar, sodass sie lediglich bei Bedarf eingeschaltet wird. Dies ist beispielsweise der Fall, wenn der erfindungsgemäße Rauchgaserzeuger in unmittelbarer Nähe des Gasmelders angeordnet ist.

Um ein Überhitzen des Prüfmediums zu vermeiden, besteht die Möglichkeit, die Heizdauer der Heizeinrichtung temporär zu begrenzen, sodass für ein erneutes Aktivieren der Heizeinrichtung ein Schalter entsprechend betätigt werden muss.

Eine weitere Lösung der Aufgabe der Erfindung sieht vor, dass ein Gaserzeuger, insbesondere ein Gaserzeuger zum Simulieren eines realen Rauchgases mit einer Sammeleinrichtung für das erzeugte Rauchgas ausgestattet ist, wobei die Sammeleinrichtung wenigstens ein Mittel zum Verschließen aufweist. Eine Sammeleinrichtung für das erzeugte Rauchgas ist deshalb vorteilhaft, da der Gaserzeuger unter anderem bedingt durch seine eingeschränkte Baugröße selten in der Lage ist, derart viel Rauchgas in einem kurzen Zeitraum zu produzieren, dass die Menge an produzierten Rauchgases meist nicht ausreicht, um einen Gasmelder zu aktivieren. Umfasst der Gaserzeuger nun eine Sammeleinrichtung, wird beispielsweise in dieser über einen längeren Zeitraum das erzeugte Rauchgas gesammelt und erst nach einem bestimmten Zeitraum in einem Vorgang freigelassen.

Um das Rauchgas zumindest solange in der Sammeleinrichtung zurückzuhalten, bis ein genügend großes Volumen an Rauchgas hergestellt ist, benötigt die Sammeleinrichtung wenigstens ein Mittel zum Verschließen der selben. Dies ist beispielsweise eine einfache Klappe oder ein Ventil oder ähnliches. Es ist hierbei nicht zwingend erforderlich, dass das Mittel zum Verschließen die Sammeleinrichtung zu 100% dicht abschließt.

Vorteilhaft ist es, wenn die Mittel zum Verschließen einen Draht aufweisen, dessen Gestalt temperaturabhängig ist. Mittels eines solchen Drahtes ist es möglich, beispielsweise die Klappe derart zu betätigen, dass sie die Sammeleinrichtung verschließt bzw. zumindest teilweise öffnet. Hierbei ist der Draht vorzugsweise der Gestalt, dass er durch einen elektrischen Strom und die damit einhergehende Temperaturerhöhung verkürzt bzw. verlängert werden kann.

Besonders vorteilhaft ist es, wenn die Mittel zum Verschließen einen Nitiuol-Draht aufweisen. Der Nitiuol-Draht zieht sich beispielsweise bei einem Durchströmen mit 2,5 Volt in Folge der daraus resultierenden Erwärmung zusammen und dehnt sich beim Abkühlen wieder entsprechend aus. Hierbei kann das Zusammenziehen des Drahtes zum Öffnen der Klappe verwendet werden.

Es versteht sich, dass alternativ hierzu verständlicherweise jeder anderer Aktor oder beispielsweise auch ein Linearmotor verwendet werden kann.

Gemäß eines Ausführungsbeispiels ist weiter vorgeschlagen, dass die Sammeleinrichtung eine Rauchzuflussöffnung aufweist. Mittels dieser Rauchzuflussöffnung ist es möglich, dass ein erzeugter Rauch beispielsweise von der Wärmekammer des Gaserzeugers in die Sammeleinrichtung gelangt.

Eine bevorzugte Ausführungsvariante sieht vor, dass die Sammeleinrichtung wenigstens eine Eintrittsöffnung und/oder wenigstens eine Austrittsöffnung aufweist.

Unter den Begriffen "Eintrittsöffnung" bzw. "Austrittsöffnung" versteht man hierbei jegliche Öffnung, durch welche beispielsweise ein Volumenstrom in die Sammeleinrichtung hinein- bzw. hinausströmen kann. Durch die Eintrittsöffnung bzw. durch die Austrittsöffnung ist gewährleistet, dass das Rauchgas an sich durch einen Unterdruck oder mittels eines weiteren Gases aus der Sammeleinrichtung herausgelangt. Es ist auch möglich, dass in der Sammeleinrichtung kurzzeitig ein Überdruck hergestellt wird, der das Rauchgas aus der Sammeleinrichtung "herausschießt".

Bevorzugt strömt durch die Eintritts- bzw. Austrittsöffnung ein Luftvolumen.

Besonders vorteilhaft ist es, wenn die Sammeleinrichtung einen Gasmelder aufweist. Um beispielsweise den Gaserzeuger hinsichtlich einer Rauchgasentwicklung zu überprüfen, ist es vorteilhaft, wenn der entsprechende Gasmelder unmittelbar in der Sammeleinrichtung des Gaserzeugers angeordnet ist. Beispielsweise sind die Eintrittsöffnung und die Austrittsöffnung im Normalbetrieb geöffnet, so dass ein Luftvolumen, welches beispielsweise in einem Klimaanlagensystem zirkuliert, die Sammeleinrichtung zumindest teilweise durchströmt. Hierdurch ist unter anderem gewährleistet, dass der Gasmelder im Normalbetrieb ebenfalls durch das Luftvolumen bzw. durch einen Teil des Luftvolumens durchströmt wird.

Unter dem Begriff "Normalbetrieb" versteht man hierbeibeispielsweise einen stationären Betrieb einer Anlage ohne Störfall.

Um den Gasmelder nun zu überprüfen, werden die Eintrittsöffnung und die Austrittsöffnung entsprechend der vorhergehend beschriebenen Techniken (Klappe, Ventil) verschlossen, so dass ein durch den Gaserzeuger erzeugtes Rauchgas nicht aus der Sammeleinrichtung entweichen kann und somit unmittelbar in Kontakt mit dem Gasmelder kommt. Nach einer erfolgreichen Überprüfung des Gasmelders werden die Eintrittsöffnung und die Austrittsöffnung der Sammeleinrichtung wieder freigegeben, so dass die Sammeleinrichtung durch den Luftvolumenstrom freigeblasen wird.

Eine vorteilhafte Weiterbildung der Erfindung sieht vor, dass die Sammeleinrichtung ein Rohr aufweist. Beispielsweise ist das Rohr der Gestalt, dass es an einem Lüftungskanal angebracht werden kann, wobei dann ein Teil des durch den Lüftungskanal strömenden Volumenstroms das an dem Lüftungskanal angeordnete Rohr durchströmt. Hierbei bildet das Rohr einen "Bypass" zu dem eigentlichen Lüftungskanal. Dies eignet sich besonders gut für ein nachträgliches Ausrüsten eines Lüftungskanals mit einer Feuermeldeanlage.

Besonders vorteilhaft ist es, wenn die Sammeleinrichtung einen veränderlichen Querschnitt aufweist. Mittels des veränderlichen Querschnittes werden beispielsweise unterschiedliche Drücke und Strömungen innerhalb der Sammeleinrichtung bewirkt, was sich positiv auf die Verbreitung des Rauchgases auswirken kann.

Eine baulich besonders einfache Variante sieht vor, dass die Sammeleinrichtung einen Diffusor aufweist. Mittels des Diffusors können in der Sammeleinrichtung bzw. in dem Rohr unterschiedliche Strömungsgeschwindigkeiten und unterschiedliche Drücke hervorgerufen werden.

Eine vorzugsweise Ausführungsform sieht vor, dass die Sammeleinrichtung eine Venturidüse aufweist. Mittels der Venturidüse können in der Sammeleinrichtung ebenfalls unterschiedliche Drücke und Strömungsgeschwindigkeiten gezielt erreicht und eingesetzt werden.

Besonders vorteilhaft ist es, wenn der Gaserzeuger im Bereich einer Querschnittserweiterung angeordnet ist. Die Querschnittserweiterung bewirkt im allgemeinen eine Verringerung der Strömungsgeschwindigkeit und eine Verringerung des Druckes am Ort der Querschnittserweiterung. Ist in einem solchen Bereich bzw. in der Nähe einer solchen Querschnittserweiterung ein Gaserzeuger angeordnet, wirkt sich das besonders auf die Rauchgasausdehnung innerhalb der Sammeleinrichtung vorteilhaft aus.

Nach einem Ausführungsbeispiel ist weiter vorgeschlagen, dass der Gaserzeuger in einem Lüftungskanal angeordnet ist. An diesem Ort lässt sich besonders effektiv ein Rauchgas erzeugen.

Ebenfalls ist nach der Erfindung vorgeschlagen, dass der Gasmelder in einem Lüftungskanal angeordnet ist.

Die Anordnung des Gaserzeugers und/oder Gasmelders in dem Lüftungskanal baulich besonders einfach zu gestalten sowie besonders platzsparend.

Die der Erfindung zugrunde liegende Aufgabe wird weiter von einem Verfahren zum Prüfen eines Gasmelders gelöst, bei dem wenigstens ein dezentral angeordneter Gaserzeuger mittels einer zentralen Überwachungseinrichtung aktiviert wird. Durch die Aktivierung erzeugt der Gaserzeuger ein Gas, vorzugsweise ein Rauchgas, welches von dem Gasmelder detektiert wird. Hierbei aktiviert sich der Gasmelder, wobei er ein entsprechendes Datensignal übermittelt.

Unter einer Überwachungseinrichtung versteht man beispielsweise eine zentrale Einrichtung in einem Gebäude, in der die sicherheitsrelevanten Funktionen des Gebäudes überwacht werden.

Besonders vorteilhaft ist es hierbei, dass ein Gasmelder nicht unmittelbar vor Ort, beispielsweise durch Personal geprüft und somit aktiviert werden muss, sondern auf einfache Art und Weise von einer zentralen Einrichtung aus geprüft bzw. aktiviert werden kann.

Eine bevorzugte Verfabrensvariante sieht vor, dass der Gasmelder das Datensignal an die zentrale Überwachungseinrichtung und/oder an eine Notfalleinrichtung übermittelt. Beispielsweise ist die zentrale Überwachungseinrichtung unmittelbar in einem Gebäude vorhanden, so dass alle zur Prüfung notwendigen Schritte von dort aus koordiniert werden können.

Vorteilhaft ist es auch, wenn das Datensignal zusätzlich oder ausschließlich an eine Notfalleinrichtung übermittelt wird. Eine Notfalleinrichtung ist beispielsweise eine ortsansässige Feuerwehr oder ein sonstiger Rettungsdienst.

Falls das Datensignal parallel zu der zentralen Überwachungseinrichtung auch an die Notfalleinrichtung übermittelt wird, ist es von Vorteil, wenn der Notfalleinrichtung vor dem Prüfen eine Information über das bevorstehende Prüfen übermittelt wird. Beispielsweise enthält die Information Daten darüber, an welchem Tag und zu welcher Uhrzeit die Prüfung stattfindet sowie über die Art der Prüfung und über die Dauer der Prüfung. Vorzugsweise wird die Notfalleinrichtung automatisch von der zentralen Überwachungseinrichtung informiert.

Es wird vorgeschlagen, dass wenigstens eine Signalleitung zwischen der zentralen Überwachungseinrichtung und der Notfalleinrichtung und/oder wenigstens eine Signalleitung zwischen wenigstens einem Gasmelder und der Notfalleinrichtung deaktiviert wird. Um die Notfalleinrichtung, beispielsweise die Feuerwehr, nicht versehentlich in einen Alarmzustand zu versetzen, ist es vorteilhaft, wenn die Datenleitung zu dieser Notfalleinrichtung zumindest temporär deaktiviert wird.

Letztlich wird vorgeschlagen, dass der Notfalleinrichtung ein Prüfungsprotokoll übermittelt wird. Ist die Prüfung abgeschlossen, ist es vorteilhaft, wenn die Notfalleinrichtung beispielsweise über den aktuellen Zustand der Gasmelder eines Gebäudes unterrichtet wird.

Die Aufgabe der Erfindung wird ebenfalls von einem Verfahren zum Prüfen eines Rauchgasmelders gelöst, bei dem ein in die Nähe des Rauchgasmelders angeordneter Rauchgaserzeuger mittels eines Prüfmediums ein Rauchgas erzeugt, und das Rauchgas eine Prüfung eines Rauchgasmelders einleitet, wobei das Prüfmedium durch eine elektrische Heizeinrichtung verraucht wird. Das Rauchgas wird hierbei von einem elektrischen Gebläse zu dem Rauchgasmelder gefördert, wobei nach der eingeleiteten Prüfung der Rauchgasmelder mittels des Gebläses gelüftet wird. Vorteilhaft bei diesem Verfahren ist es, dass erfindungsgemäß das Rauchgas durch eine elektrische Heizeinrichtung erzeugt wird, in dem ein Prüfmedium durch die elektrische Heizeinrichtung verraucht wird. Hierbei ist es besonders vorteilhaft, dass für die Erzeugung des Rauchgases nur eine sehr geringe Menge eines Prüfmediums verbraucht wird.

Vorteilhaft ist es auch, dass das Rauchgas durch ein elektrisches Gebläse des Rauchgaserzeugers unmittelbar in bzw. an den Rauchgasmelder geblasen wird, sodass ein gezieltes Berauchen des Rauchgasmelders geschieht, wobei unter anderem durch die Zielstrebigkeit des Verfahren die Effektivität einer Rauchgasmelderprüfung wesentlich erhöht wird.

Ist die Prüfsequenz des Rauchgasmelders erfolgreich eingeleitet, ist es besonders vorteilhaft, wenn der Rauchgasmelder unmittelbar nach dem Berauchen nachgelüftet wird, sodass die Prüfung schnellstmöglich beendet wird. Durch das gezielte Nachbelüften mittels des erfindungsgemäßen Rauchgaserzeugers wird die Gefahr einer unerwünschten Ablagerung von Rauchgaspartikeln in dem Rauchgasmelder verringert.

Es versteht sich, dass die vorhergehend beschriebenen Gaserzeuger und Hitzeerzeuger, insbesondere die Rauchgaserzeuger, vorzugsweise mit all ihren beschriebenen vorteilhaften Merkmalen nicht nur stationär angeordnet sein können, sondern ebenfalls auch mobil einsetzbar sind. Hierzu ist es lediglich notwendig, dass sie in eine entsprechende Prüfeinrichtung eingesetzt werden.

Ein Ausführungsbeispiel einer erfindungsgemäßen Rauchgasmeldeanlage und Ausführungsbeispiele verschiedener Rauchgaserzeuger sind in der Zeichnung dargestellt und werden im Folgenden näher erläutert. Es zeigt
- Figur 1: eine Rauchgasmeldeanlage aus Rauchgasdetektor und Rauchgaserzeuger,
- Figur 2: einen Rauchgaserzeuger mit einem erwärmbaren benetzbaren Stab,
- Figur 3: einen Rauchgaserzeuger mit einem beheizbaren Kapillarrohr,
- Figur 4: einen Rauchgaserzeuger mit Rauchgaspatronen,
- Figur 5: einen Rauchgaserzeuger mit einem Flüssigkeitssprühbehälter,
- Figur 6 bis 9: unterschiedlichste Ausführungsbeispiele von Hitzemeldern und entsprechenden Hitze- erzeuger,
- Figur 10: eine zweigeteilte Wärmekammer,
- Figur 11: eine teilweise geschnittene Wärmekammer,
- Figur 12: schematisch eine Akku-Stromquelle,
- Figur 13: einen Widerstand und eine Manschette mit Kapillarflächen,
- Figur 14: eine schematische Darstellung eines erfindungsgemäßen Rauchgaserzeugers,
- Figur 15: einen alternativen Rauchgaserzeuger in Wechselwirkung mit einem Rauchgasmelder,
- Figur 16 und 17: einen Rauchgasmelder in einem Lüftungskanal,
- Figur 18 und 19: einen alternativen Rauchgasmelder in einem Lüftungskanal,
- Figur 20: einen weiteren Rauchgasmelder in einem Bypass eines Lüftungskanals,
- Figur 21: einen Rauchgasmelder in einem alternativen Bypass eines Lüftungskanals und
- Figur 22: ein Verfahren zum Prüfen wenigstens eines Gasmelders.

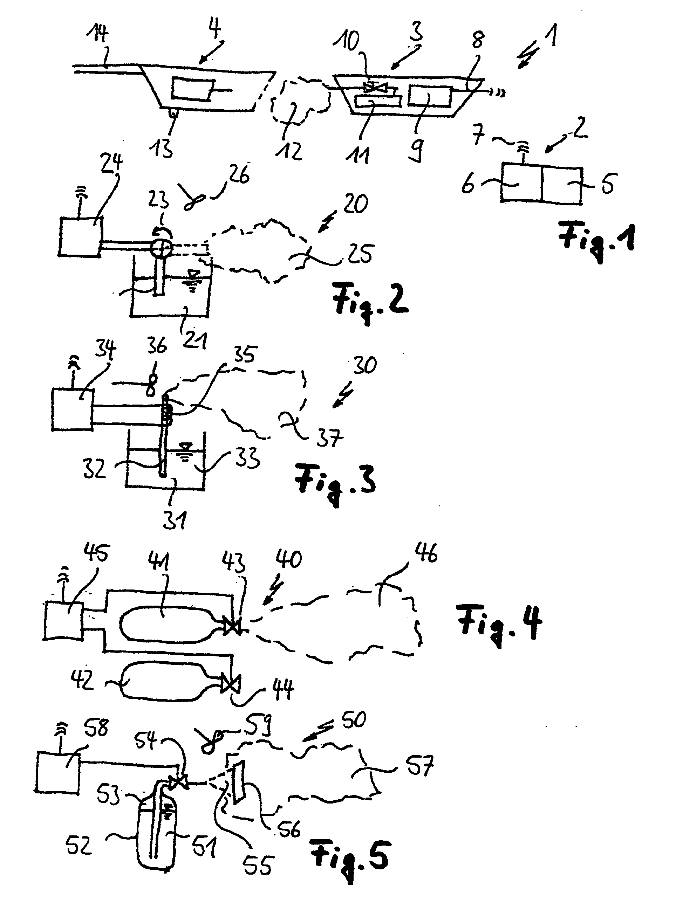

Die in Figur 1 dargestellte Rauchgasmeldeanlage 1 besteht aus dem Sender 2, dem Rauchgaserzeuger 3 und dem Rauchgasdetektor 4. In dieser Anordnung sind der Rauchgaserzeuger 3 und der Rauchgasdetektor 4 derart zueinander fixiert, dass sie miteinander in Wirkverbindung stehen.

Der Sender 2 besteht aus einer Zeituhr 5 und einer Sendeeinrichtung 6. Die Zeituhr 5 erlaubt es somit, zu bestimmten vorgegebenen Zeiten wie beispielsweise jeden Monat die Sendeanlage 6 auszulösen, so dass diese ein Signal 7 abgibt. Dieses Signal 7 wird von der Antenne 8 des Rauchgaserzeugers aufgenommen und die Empfangsanlage 9 bewirkt ein Öffnen des Ventils 10, so dass Rauchgas aus dem Reservoir freigegeben wird. Die dabei entstehende Rauchgaswolke 12 dehnt sich aus und wird von dem in der Nähe angeordneten Detektor 4 erfasst. Der Detektor 4 lässt im Detektionsfall eine Leuchtdiode 13 blinken und gibt über die Leitung 14 ein Steuersignal ab. Mit diesem Steuersignal kann beispielsweise ein Rolltor geschlossen werden oder eine Sprinkleranlage angeschaltet werden.

Die Sendeanlage 2 ist leicht zugänglich angeordnet, so dass sie auch manuell bedienbar ist. Der Rauchgaserzeuger 3 und der Rauchgasdetektor 4 sind vorzugsweise im Deckenbereich von Gebäuden nahe beieinanderliegend angeordnet, so dass sichergestellt ist, dass die Rauchgaswolke 12 vom Rauchgasdetektor 4 wahrgenommen wird.

Je nach Anwendungsfall kann anstelle eines Rauchgases auch jedes andere Gas erzeugt werden, um einen entsprechenden Gasdetektor 4 auf dessen Wirksamkeit zu prüfen.

Die Figuren 2 bis 5 zeigen verschiedene Ausführungsbeispiele von Gaserzeugern.

Der in Figur 2 gezeigte Rauchgaserzeuger 20 besteht aus einem Flüssigkeitsreservoir 21, in das ein Stab 22 eingetaucht ist. Der Stab 22 ist entsprechend dem Pfeil 23 drehbar gelagert, so dass er um 45° in die gestrichelt dargestellte Position geschwenkt werden kann, wenn mittels des Empfängers 24 ein Funksignal wahrgenommen wird. Durch das Schwenken des Stabes 22 wird der Stab 22 derart mit einer Stromquelle verbunden, dass er sich erhitzt und die an ihm haftende Flüssigkeit verdampft. Die dabei entstehende Rauchgaswolke 25 wird mittels des Gebläses 26 in Richtung zum Rauchgasdetektor (nicht gezeigt) geblasen.

Eine alternative Ausführungsform eines Rauchgaserzeugers 30 ist in Figur 3 dargestellt. Bei diesem Rauchgaserzeuger wird aus einem Reservoir 31 mittels eines Kapillarrohres 32 Flüssigkeit 33 angesaugt. Der Empfänger 34 bewirkt, dass bei einem empfangenen Signal die Heizwendel 35 von Strom durchflossen wird und einen Teil des angesaugten Fluids 33 verdampft. Das Gebläse 36 sorgt dafür, dass die entstehende Rauchgaswolke 37 zu einem Detektor (nicht gezeigt) geblasen wird.

Die Figur 4 zeigt einen Gaserzeuger 40, der es erlaubt, zwei unterschiedliche Gasarten freizugeben. Hierzu sind zwei Gasflaschen 41, 42 vorgesehen, deren Auslässe Ventile 43, 44 aufweisen, die vom Empfänger 45 je nach empfangenem Signal geöffnet werden. Dadurch entsteht eine Gaswolke 46, die von einem Detektor (nicht gezeigt) wahrgenommen werden kann. Dieser Rauchgaserzeuger ermöglicht es somit, zu testen, ob der Detektor auch auf unterschiedliche Gasarten anspricht.

Ein weiteres Ausführungsbeispiel eines Rauchgaserzeugers 50 ist in Figur 5 gezeigt. Bei dieser Einrichtung ist eine mit einer Flüssigkeit 51 gefüllte Druckflasche 52 vorgesehen. Ein unter Druck stehendes Gaspolster 53 sorgt dafür, dass beim Öffnen des Ventils 54 ein Sprühstrahl 55 freigesetzt wird. Dieser Sprühstrahl trifft auf eine beheizte Fläche 56, so dass das aufgesprühte Fluid verdampft und eine Gaswolke 57 erzeugt. Ein Empfänger 58 ist so mit dem Ventil 54 verschaltet, dass bei einem Empfang eines Signals das Ventil 54 geöffnet wird und ein Sprühstrahl auf die Platte 56 trifft und dort verdampft. Das Gebläse 59 bläst die Gaswolke 57 zu einem Detektor (nicht gezeigt) um dort ein Signal auszulösen.

Die dargestellten Ausführungsbeispiele zeigen, dass verschiedene Gaserzeuger möglich sind, um auf einfache Art und Weise ein Gas zu erzeugen, das von einem Gasmelder wahrgenommen werden kann, um ein Signal auszulösen. Der Fachmann erkennt, dass die Möglichkeiten der Rauchgaserzeugung nicht auf die angegebenen Ausführungsbeispiele beschränkt sind.

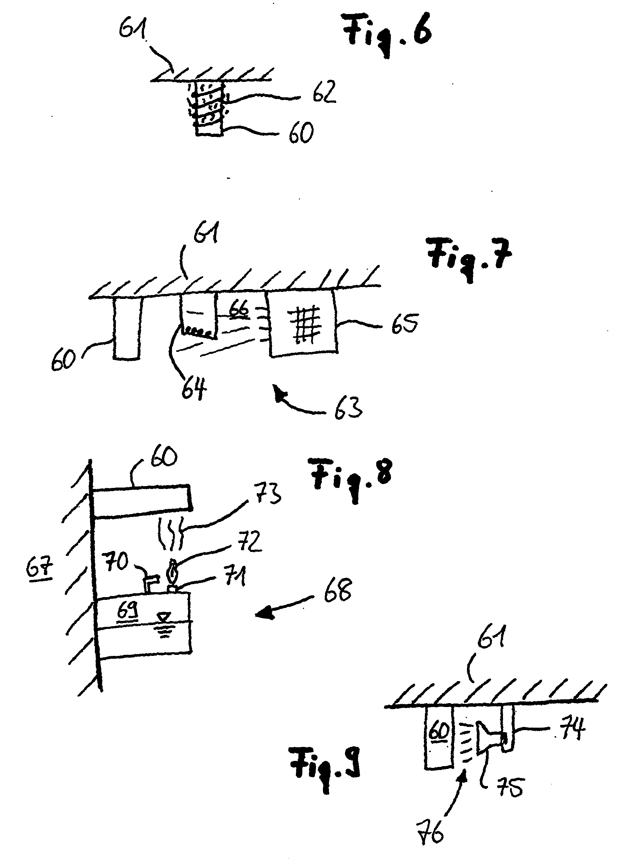

Der in Figur 6 dargestellte Hitzemelder 60 ist an einer Gebäudedecke 61 angeordnet und bildet eine körperliche Einheit mit einem Hitzeerzeuger 62, welcher in Gestalt eines Metalldrahtes 62 dargestellt ist. Soll der Hitzemelder 60 nun getestet werden, wird durch den Metalldraht 62 ein elektrischer Strom geleitet, wobei sich der Metalldraht 62 erhitzt und die erzeugte Hitze ausreicht, um den Hitzemelder 60 zu aktivierten.

Der in Figur 7 gezeigte Hitzemelder 60 ist ebenfalls an der Decke 61 angeordnet. In der Nähe des Hitzemelders 60 ist eine Hitzeerzeugungsanlage 63 angeordnet, wobei die Hitzeerzeugungsanlage 63 aus einer Heizwendel 64 und einem Gebläse 65 besteht. Bei einem Test des Hitzemelders 60 wird die Glühwendel 64 aktiviert, sodass die Glühwendel 64 Hitze erzeugt. Diese Hitze wird dann mittels des Gebläses 65 und einer Luftströmung 66 an den Hitzemelder herangetragen, sodass diese die Hitze detektiert.

Ebenfalls ist es möglich, den Hitzemelder 60 an einer vertikal verlaufenden Fläche 67 anzuordnen. Unter dem Hitzemelder 60 befindet sich ein Hitzeerzeuger 68, wobei der Hitzeerzeuger 68 einen Fluidbehälter 69 sowie eine Zündeinrichtung 70 aufweist. Durch eine Düse 71 strömt hierbei ein brennbares Medium, welches durch die Zündeinrichtung 70 entzündet wird, sodass ein Flamme 72 eine Hitze 73 entwickelt, wobei die aufsteigende Hitze 73 dem Hitzemelder 60 auslöst.

In einem weiteren Ausführungsbeispiel ist der Hitzemelder 60 wiederum an der Decke 61 angeordnet. In unmittelbarer Nähe des Hitzemelders 60 ist ein Hitzeerzeuger 74 angeordnet. Der Hitzeerzeuger 74 weist einen Wärmestrahler 75 in Form einer Infrarotlampe auf. Ist die Infrarotlampe aktiviert, erzeugt sie eine Wärmestrahlung 76, die den Hitzemelder 60 aktiviert.

Es versteht sich, dass die vorhin beschriebenen Möglichkeiten hinsichtlich eines Hitzemelders und Hitzeerzeugers nicht auf die angegebenen Ausführungsbeispiele beschränkt sind, sondern dass es eine Vielzahl von weiteren Ausführungsbeispielen geben kann. Hieraus ergibt sich, dass die Erfindung auf jeden beliebigen Hitzedecktor anwendbar ist, in dem ein entsprechender Hitzeerzeuger vorgesehen wird. Hierbei sind der Hitzemelder und der Hitzeerzeuger entweder derart zu einander fixiert, dass sie als Anordnung miteinander in Wirkverbindung stehen oder der Hitzeerzeuger bildet mit dem Hitzemelder eine körperliche Einheit.

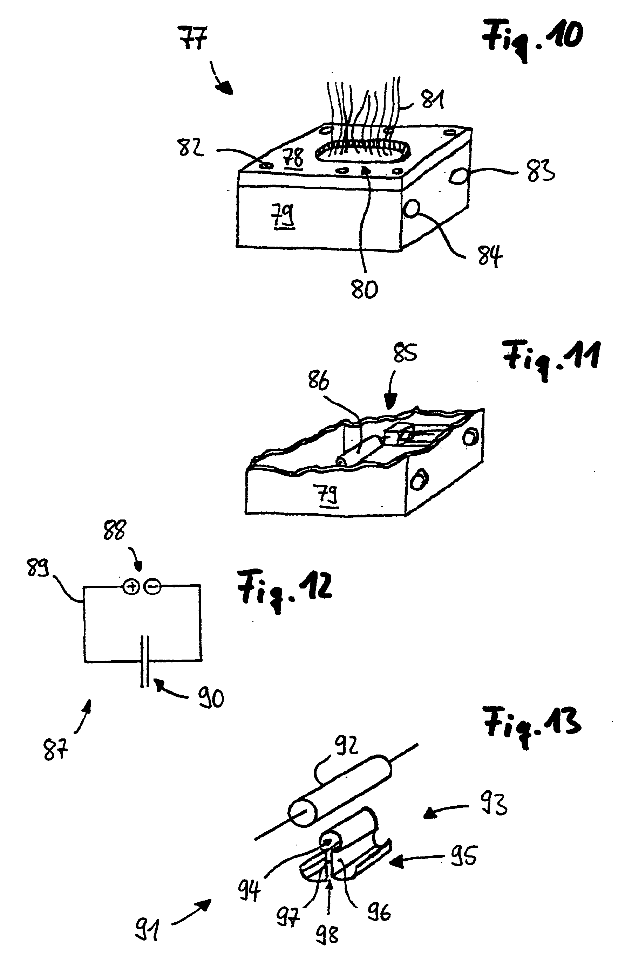

Die in Figur 10 dargestellte zweigeteilte Wärmekammer 77 weist einen Verschlussdeckel 78 und ein Basisgehäuse 79 auf. Der Verschlussdeckel 78 hat in seiner Mitte eine Öffnung 80, durch welche ein erzeugtes Rauchgas 81 aufsteigt. Des Weiteren ist der Verschlussdeckel 78 mittels einer Vielzahl von Schrauben 82 mit dem Basisgehäuse 79 verschraubt. Das Basisgehäuse 79 hat an einer seiner Seiten zwei Bohrungen 83 und 84, welche Steckkontakte für einen elektrischen Anschluss (hier nicht dargestellt) aufnehmen.

Im Inneren des Basisgehäuses 79 ist eine Heizeinrichtung 85 angeordnet, wobei die Heizeinrichtung 85 einen Widerstand 86 aufweist.

In der Figur 12 ist prinzipiell der einfache Aufbau eines elektrischen Stromkreises 87 des erfindungsgemäßen Rauchgaserzeugers dargestellt. Als Stromquelle dient hierbei ein Akkumulator 88, welcher mittels einer Drahtverbindung 89 mit einem Wärmeleitkörper 90 (Kondensator, ohmischer Widerstand) verbunden ist.

Die Anordnung 91 der Figur 13 zeigt einen ohmschen Widerstand 92 sowie eine Kapillarmanschette 93. Der Außendurchmesser des ohmschen Widerstandes 92 entspricht in etwa dem Innendurchmesser der Kapillarmanschette 93 in einem Bereich 94 mit seinem unteren Bereich 95 ist die Kapillarmanschette 93 in einem gelartigen Prüfmedium (hier nicht dargestellt) angeordnet, wobei sich das gelartige Prüfmedium im Innenbereich zwischen den Kapillarflächen 96 und 97 mittels Kapillarkräfte in Pfeilrichtung 98 zwischen den beiden Kapillarinnenflächen 96 und 97 zu dem ohmschen Widerstand 92 hinbewegt.

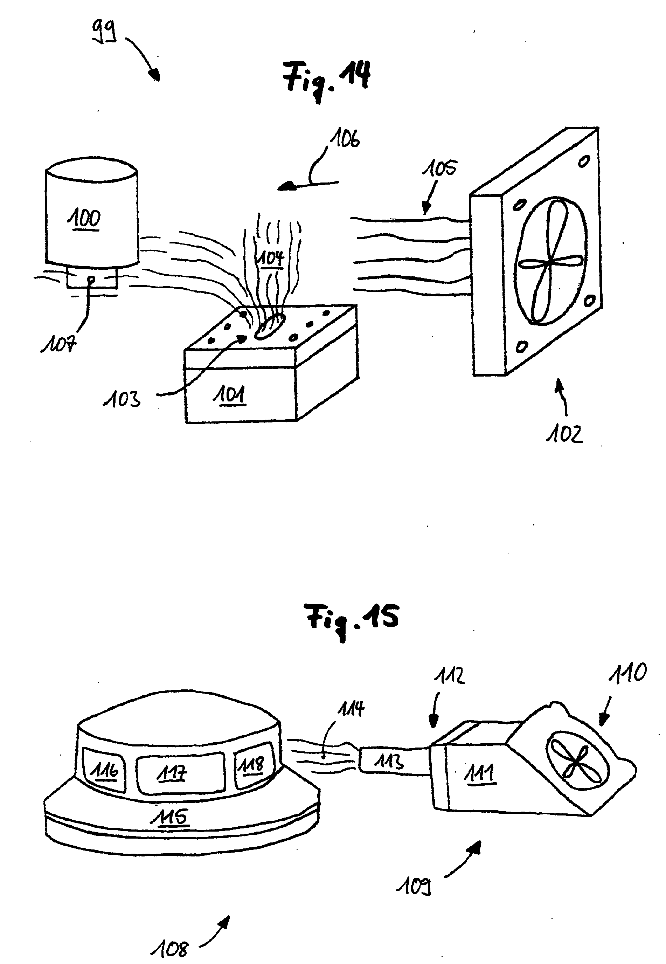

Eine Anordnung 99 der Figur 14 umfasst einen Rauchgasmelder 100, eine Wärmekammer 101 und einen Ventilator 102. Durch eine Öffnung 103 der Wärmkammer 101 gelangt ein Rauchgas 104 in die unmittelbare Umgebung der Anordnung 99. Der Ventilator 102 bläst einen Luftstrom 105 in Pfeilrichtung 106. Hierbei wird das Rauchgas 104 mit der Luftströmung 105 mitgeführt und von einem Detektor 107 des Rauchgasmelders 100 registriert, wodurch ein Alarmsignal injiziert wird.

Ist die Prüfung des Rauchgasmelders 100 abgeschlossen, wird die Entwicklung des Rauchgases 104 in der Wärmekammer 101 unterbunden, in dem die elektrische Heizeinrichtung 85 ausgeschaltet wird. Der Luftstrom 105 des Ventilators 102 bläst dem Rauchgasmelder 100, insbesondere den Detektor 107 des Rauchgasmelder 100, von den restlichen Rauchgaspartikeln des Rauchgases 104 frei.

In dem Ausführungsbeispiel 108 ist eine Rauchgaserzeuger 109 und ein Ventilator 110 in einem Gehäuse 111 zusammen angeordnet. Das Gehäuse 111 weist in seinem vorderen Bereich 112 ein Rohr 113 auf, durch welches ein durch den Rauchgaserzeuger 109 erzeugtes Rauchgas 114 mittels des Ventilators 110 geblasen wird. Das Gehäuse 111 ist hierbei derart relativ zu einem Rauchgasmelder 114 angeordnet, dass das Rauchgas 114 unmittelbar über Öffnungen 116, 117 und 118 bis an den Detektor des Rauchgasmelders 115 gelangt. Mittels des Rohres 113 wird ein gezieltes Anströmen des Rauchgasmelders 115 durch Rauchgas 114 des Rauchgaserzeugers 109 auf einfache Art und Weise möglich.

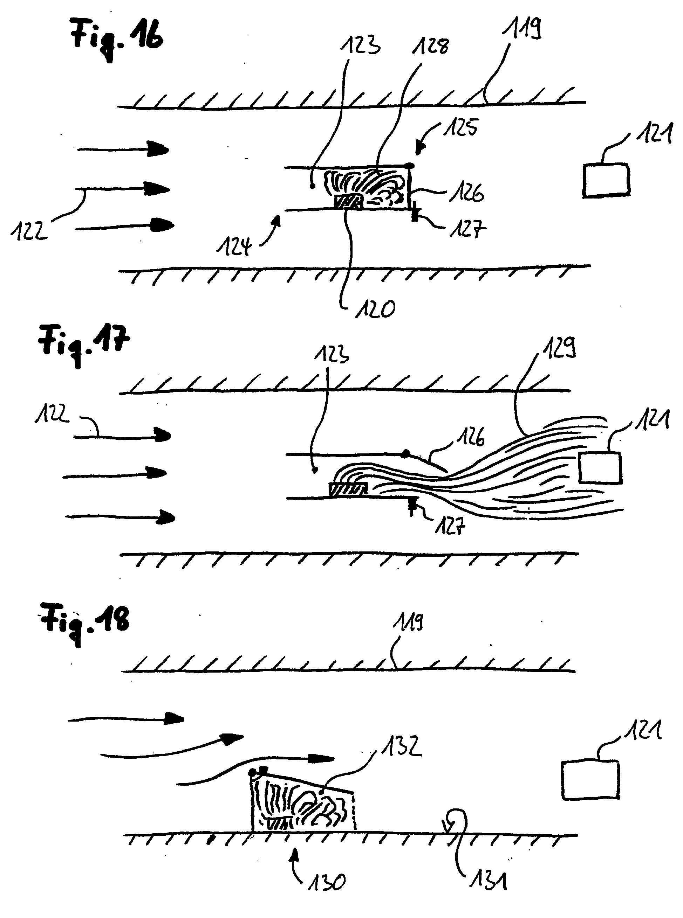

Der Lüftungskanal 119 der Figuren 16 bis 19 weist einen Rauchgaserzeuger 120 und einen Rauchgasmelder 121 auf. Der Rauchgaserzeuger 120 und der Rauchgasmelder 121 sind jeweils auf der Mittelachse des Lüftungskanals 119 hintereinander angeordnet. Durch den Lüftungskanal 119 strömt ein Luftvolumen 122. Das Luftvolumen 122 trifft zuerst auf den Rauchgaserzeuger 120 und anschließend auf den Rauchgasmelder 121.

Der Rauchgaserzeuger 120 umfasst eine Rauchgassammelkammer 123, die auf der Rauchgasmelder 121 abgewandten Seite 124 nicht verschlossen ist. An der dem Rauchgasmelder 121 zugewandten Seite 125 weist die Rauchgassammelkammer 123 einen Verschluss 126 auf. In Bereich des Verschlusses 126 befindet sich ein Verschluss 127, der vorzugsweise über einen elektrischen Impuls es ermöglicht, dass die Klappe 126 geöffnet wird.

Soll nun der Rauchgasmelder 121 geprüft werden, wird der Rauchgaserzeuger aktiviert, sodass er ein Rauchgas 128 produziert. Dieses Rauchgas 128 sammelt sich solange in der Rauchgassammelkammer 123 bis die Klappe 126 mittels des Verschlusses 127 geöffnet wird und ein konzentrierter Rauchgaszug 129 aus der Rauchgassammelkammer 123 in Richtung des Rauchgasmelders 121 entweicht. Der Rauchgaszug 129 wird mit dem Volumenstrom 122 durch den Lüftungskanal 119 mitgezogen.

Erfindungsgemäß kann sich in der Rauchgassammelkammer 123 über einen genügend langen Zeitraum derart viel Rauchgas 128 ansammeln, sodass das angesammelte Rauchgas 128 einen derart starken Rauchgaszug 129 in den Lüftungskanal 119 bewirkt, dass die Detektoren des Rauchgasmelders 121 auf diesem Rauchgaszug 129 ansprechen.

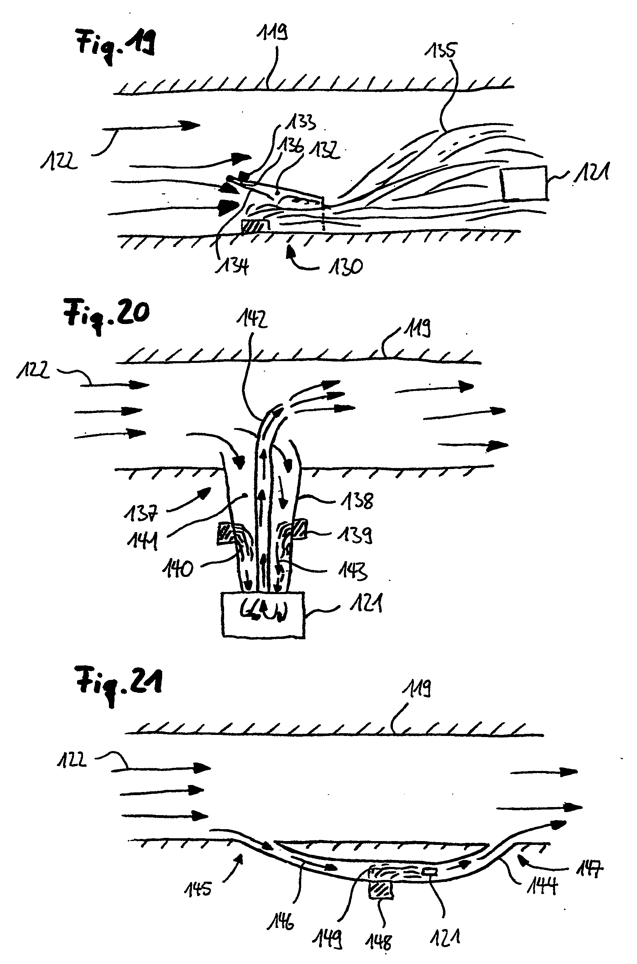

In dem Lüftungskanal 119 der Figuren 18 und 19 ist ein alternativer Rauchgaserzeuger 130 angeordnet, der bei Bedarf mit dem Rauchgasmelder 121 wechselwirkt. Der Rauchgasmelder 130 ist in diesem Ausführungsbeispiel nicht auf der Mittelachse des Lüftungskanals 119 angeordnet, sondern an der Innenseite 133 angeordnet. Der alternative Rauchgasmelder umfasst ebenfalls einen etwas größeren Hohlraum als Rauchgassammelkammer 132, in dem sich bei aktiviertem Rauchgaserzeuger 130 ein entsprechendes Rauchgas ansammelt. Hat sich in der Rauchgassammelkammer 132 genügend Rauchgas angesammelt wird mittels eines Verschlusses 133 eine Klappe 134 des Rauchgasmelders 130 geöffnet, sodass ein Volumenstrom 122 die Rauchgassammelkammer 132 zumindest teilweise durchströmen kann und hierbei das Rauchgas zu einer Rauchgaspfanne 135 mit zu dem Rauchgasmelder 121 mitreist. Der Verschluss 133 wird hierbei elektrisch angesteuert, wobei sich ein Nitiuol-Draht 136 zusammenzieht und dabei die Klappe 134 öffnet.

Der in Figur 20 dargestellte Lüftungskanal 119 weist in einem Bereich einen Durchbruch 137 auf. An dem Durchbruch 137 ist ein erstes Rohr 138 angeordnet, welches eine Verbindung zwischen dem Lüftungskanal 119 und einem Rauchgasmelder 121 bildet. An dem äußeren Rohr 138 ist ein Rauchgaserzeuger 139 angeordnet, der bei Bedarf ein Rauchgas 140 herstellen kann. Im Inneren 141 des ersten äußeren Rohres 138 befindet sich ein weiteres Rohr 142, welches ebenfalls eine Verbindung zwischen dem Lüftungskanal 119 und dem Rauchgasmelder 121 bildet.

Durch den Lüftungskanal 119 strömt ein Luftvolumen 122. Das Luftvolumen 123 wird teilweise durch das Innere 141 des Rohres 138 zu dem Rauchgasmelder 121 geleitet. Von dort aus gelangt das durch das erste äußere Rohr 138 geleitete Luftvolumen mittels des zweiten inneren Rohres 142 wieder zurück in den Lüftungskanal. Da nun immer ein Teil des Luftvolumens 122 durch den Rauchgasmelder 121 mittels der beiden Rohre 138 und 142 geleitet wird, ist der Rauchgasmelder in der Lage ein Signal zu erzeugen, wenn in dem Luftvolumen 122 ein Rauchgas 140 vorhanden ist.

Soll der Rauchgasmelder 121 nun überprüft werden, wird der Rauchgaserzeuger 139 derart eingestellt, dass er ein Rauchgas 140 erzeugt. Dieses Rauchgas 140 wird nun durch ein Teilvolumen 143 des Luftvolumens 122 mittels des äußeren ersten Rohres 138 zu dem Rauchmelder 121 geführt. Dieser ist nun in der Lage das Rauchgas 140 zu detektieren und erzeugt ein entsprechendes Signal, welches ein Vorhandensein von Rauchgas 140 anzeigt.

Der Lüftungskanal 119 der Figur 21 weist einen Bypass 144 auf. Der Bypass 144 hat eine Eintrittsöffnung 145, die einen Teilvolumenstrom 146 des Volumenstroms 122 erlaubt in den Bypass 144 einzutreten und einen Auslassbereich 147, durch den der Teilvolumenstrom 146 wieder zurück in den Lüftungskanal 119 strömen kann. Der Bypass 144 weist darüber hinaus einen Rauchgaserzeuger 148 und einen Rauchgasmelder 121 auf. Des Weiteren ist der Bypass dergestalt, dass er im Bereich des Rauchgaserzeugers 148 einen größeren Querschnitt aufweist als in Bereich der Eingangsöffnung 145. Hierdurch verlangsamt sich die Strömungsgeschwindigkeit des Teilvolumenstroms 146 im Bereich des Rauchgaserzeugers 148, wodurch in diesem Bereich ein Unterdruck entsteht. Dieser bewirkt, dass ein durch den Rauchgaserzeuger 148 erzeugtes Rauchgas 149 in den Bypass 144 gesogen wird und mit dem Teilvolumenstrom 146 zu dem Rauchgasmelder 121 getragen wird.

Der Bypass 144 ist erfindungsgemäß derart an den Lüftungskanal 119 angeordnet, dass immer ein Teilvolumenstrom 146 des Volumenstroms 122 durch ihn strömt. Führt der Volumenstrom 122 ein Rauchgas mit sich, gelangt dieses mittels des Bypasses 144 zu dem Rauchgasmelder 121, der dann ein entsprechendes Signal auslöst. Soll hingegen ein Rauchgas 149 nur simuliert werden, um den Rauchgasmelder 121 auf seine Funktionstüchtigkeit hin zu überprüfen, wird das Rauchgas 149 mittels des Rauchgaserzeugers 148 erzeugt und mit dem Teilvolumenstrom 146 an den Rauchgasmelder 121 herangetragen.

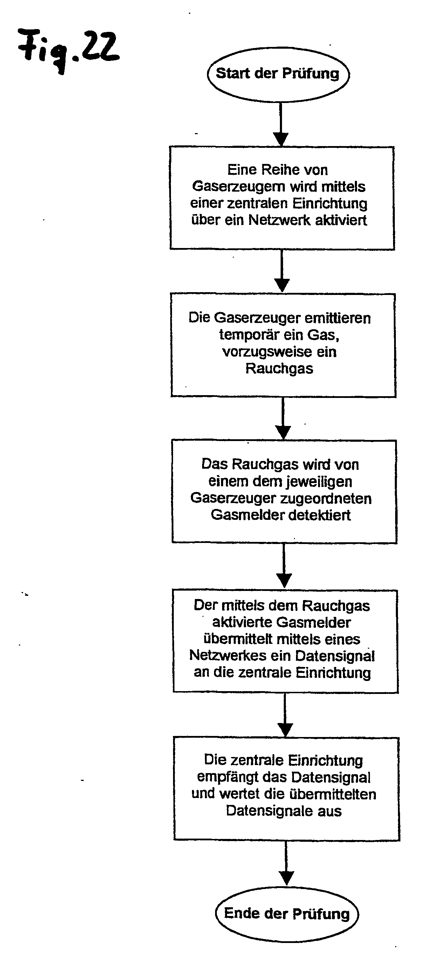

Der in der Figur 22 dargestellte Verfahrensablauf beginnt mit dem Start der Prüfung, wobei eine Reihe von Gaserzeugern mittels einer zentralen Einrichtung mittels eines Netzwerkes aktiviert wird. Daraufhin emittieren die aktivierten Gaserzeuger zumindest temporär ein Gas, wobei das Gas vorzugsweise ein Rauchgas ist. Das Rauchgas wird hierbei dem Gasmelder derart zugeführt, dass das Rauchgas von einem dem jeweiligen Gaserzeuger zugeordneten Gasmelder detektiert wird. Der mittels des Rauchgases aktivierte Gasmelder übermittelt mittels eines Netzwerkes ein Datensignal an die zentrale Einrichtung. Das Übermitteln des Datensignals zeigt an, dass der Gaserzeuger funktionsfähig ist und auf das Rauchgas anspricht. Die das Datensignal empfangene zentrale Einrichtung wertet die übermittelten Datensignale aus. Hiermit ist das Ende der Prüfung erreicht.

Erreicht ein Datensignal eines Gasmelders nicht wie vorgesehen die zentrale Einrichtung, wird der Gasmelder sowie der Gaserzeuger manuell überprüft.

Alternativ zu dem vorhergehend beschriebenen Verfahrensablauf, ist es möglich, dass ein Datensignal parallel zu der zentralen Einrichtung ebenfalls an eine weitere Einrichtung übermittelt wird. Diese Einrichtung ist beispielsweise eine Feuerwehrdienststelle. Besteht eine solche direkte Verbindung zu der Feuerwehr, ist es vorteilhaft, wenn die Feuerwehr vorzugsweise automatisch von der zentralen Einrichtung über den Zeitplan und den Ablauf der Prüfung informiert wird. Gegebenenfalls ist es möglich, dass die zentrale Einrichtung die direkte Datenleitung zu der Feuerwehr temporär deaktiviert, sodass der Feuerwehr während der Prüfung kein Datensignal übermittelt wird und somit beispielsweise die Gefahr eines Fehlalarms vermieden ist.

Nach der Prüfung wird die direkte Datenverbindung zu der Feuerwehr vorzugsweise automatisch wieder hergestellt.

Die Erfindung wurde am Beispiel eines Rauchgaserzeugers und eines Rauchgasdetektors erläutert. Sie ist jedoch auf jeden beliebigen Gasdetektor oder Hitzedetektor anwendbar, indem ein entsprechender Gaserzeuger oder Hitzeerzeuger vorgesehen wird.

## Patentansprüche

1. Erfassungssystem für ein Gas und/oder Rauchgas, welches das Folgende umfasst:
- einen Detektor zum Erfassen der Gegenwart des Gases und/oder Rauchgases, wobei der Detektor ein Gehäuse umfasst, welches mindestens eine Öffnung zur Aufnahme des Gases und/oder Rauchgases durch diese hindurch aufweist und an einer Oberfläche befestigt ist;
- eine Prüfeinrichtung zum Prüfen des Detektors, wobei die Prüfeinrichtung eine Quelle eines Prüfmediums, eine Heizeinrichtung zum Erwärmen des Prüfmediums, um ein Gas und/oder Rauchgas zu erzeugen ein Mittel zum Zuführen des Prüfmediums von der Quelle zu der Heizeinrichtung und ein Mittel zum Zuführen des Gas und/oder Rauchgases zu dem Detektor umfasst, wobei die Prüfeinrichtung im Gebrauch so angeordnet ist, dass das Gas und/oder Rauchgas auf die Öffnung geleitet wird, um den Detektor zu testen.
- **dadurch gekennzeichnet, dass** die Prüfeinrichtung in einer festen Position neben dem Detektorgehäuse an der Oberfläche befestigt ist und eine Rauchgaswolke in Richtung Detektor abgibt.

2. System nach Anspruch 1, wobei der Detektor und die Prüfeinrichtung eine körperliche Einheit bilden.

3. System nach Anspruch 1, wobei das Mittel zum Zuführen des Gas und/oder Rauchgases zu dem Detektor ein Gebläse umfasst, um den Strom des Gas und/oder Rauchgases von der Prüfeinrichtung zu dem Detektor zu leiten.

4. System nach Anspruch 1, wobei das Mittel zum Zuführen des Gas und/oder Rauchgases zu dem Detektor ein Rohr oder einen Schlauch umfasst, um den Strom des Gas und/oder Rauchgases von der Prüfeinrichtung zu dem Detektor zu leiten.

5. System nach Anspruch 1, wobei es sich bei dem Prüfmedium um eine Flüssigkeit, ein Gel oder einen Feststoff handelt.

6. System nach Anspruch 1, wobei das Prüfmedium ein Material umfasst, welches in ein Gas und/oder Rauchgas umgewandelt wird, wenn es erwärmt wird.

7. System nach Anspruch 1, wobei die Heizeinrichtung elektrisch betrieben wird.

8. System nach Anspruch 3, wobei das Gebläse so eingerichtet ist, dass es weiter Luft in Richtung auf den Detektor bläst, nachdem es dem Detektor das Gas und/oder Rauchgas zugeführt hat.

9. System nach Anspruch 1, wobei die Prüfeinrichtung ferner eine Batterie oder eine wiederaufladbare Batterie umfasst, um die Prüfeinrichtung mit Spannung zu versorgen.

10. System nach Anspruch 1, welches ferner ein Spannungsversorgungsmittel umfasst, um Netzspannung oder Niederspannung aus einer anderen Spannungsquelle bereitzustellen.

11. System nach Anspruch 1, welches ferner ein Steuermittel umfasst, um die Aktivierung und Deaktivierung der Prüfeinrichtung zu steuern.

12. System nach Anspruch 11, wobei sich das Steuermittel in drahtgebundenem Datenaustausch mit der Prüfeinrichtung befindet.

13. System nach Anspruch 11, wobei das Steuermittel entfernt von der Prüfeinrichtung angeordnet ist und so eingerichtet ist, dass es die Aktivierung und Deaktivierung der Prüfeinrichtung über einen drahtlosen Datenaustausch steuert.

14. System nach Anspruch 1, wobei die Prüfeinrichtung eine Heizkammer umfasst, welche auf deren einer Seite ein Sperrelement umfasst, das derart angeordnet ist, dass verhindert wird, dass ein von der Prüfeinrichtung erzeugtes Gas und/oder Rauchgas in Richtung auf den Detektor geleitet wird, wenn sich das Sperrelement in der geschlossenen Position befindet.

15. System nach Anspruch 14, wobei die Heizkammer ferner ein Verschlusselement umfasst, welches so angeordnet ist, dass es die Position des Sperrelements steuert.

16. Erfassungssystem für ein Gas und/oder Verbrennungsprodukt, welches das Folgende umfasst:
- einen Lüftungskanal;
- einen Detektor zum Erfassen der Gegenwart des Gases und/oder Verbrennungsproduktes in dem Kanal; und
- eine Prüfeinrichtung zum Prüfen des Detektors, umfassend:
- eine Quelle eines Prüfmediums;
- eine Heizeinrichtung zum Erwärmen des Prüfmediums, um ein Gas und/oder Rauchgas zu erzeugen;
- ein Mittel zum Zuführen des Prüfmediums von der Quelle zu der Heizeinrichtung; und
- ein Mittel zum Zuführen des Gas und/oder Rauchgases zu dem Detektor,
- wobei die Prüfeinrichtung in einer festen Position neben dem Detektor auf dem Lüftungskanal angeordnet ist.

17. System nach Anspruch 16, wobei sowohl der Detektor als auch die Prüfeinrichtung in dem Lüftungskanal angeordnet sind.

18. System nach Anspruch 16, wobei die Prüfeinrichtung in dem Lüftungskanal angeordnet ist.

19. System nach Anspruch 16, wobei die Prüfeinrichtung auf der Leitung außerhalb des langgestreckten Kanals angeordnet ist und das Mittel zum Zuführen des Gas und/oder Rauchgases zu dem Detektor ein Rohr oder einen Schlauch umfasst, um den erzeugten Reiz in den Lüftungskanal zu leiten.

20. System nach Anspruch 16, wobei die Prüfeinrichtung eine Heizkammer umfasst, welche auf deren einer Seite ein Sperrelement umfasst, das derart angeordnet ist, dass verhindert wird, dass ein von der Prüfeinrichtung erzeugtes Gas und/oder Rauchgas in Richtung auf den Detektor geleitet wird, wenn sich das Sperrelement in der geschlossenen Position befindet.

21. System nach Anspruch 14, wobei die Heizkammer ferner ein Verschlusselement umfasst, welches so angeordnet ist, dass es die Position des Sperrelements steuert.

## Claims

1. A detection system for a gas and/or combustion product comprising:
a detector for detecting the presence of the gas and/or combustion product, the detector comprising a housing having at least one aperture for receiving the gas and/or combustion product therethrough and fixed to a surface;
a testing device for testing the detector, the device comprising a source of test media, a heating means for heating the test medium in order to generate test stimulus representative of the gas or combustion product, means for supplying the test medium to the heating means from the source; and means for supplying the test stimulus to the detector whereby in use the testing device is arranged to direct the test stimulus toward the aperture in order to test the detector,
**characterized in that** the device is mounted to the surface in a fixed position adjacent to the detector housing and a gas cloud is emitted toward the detector.

2. The system of claim 1 wherein the detector and the testing device form one physical unit.

3. The system of claim 1 wherein the means for supplying the test stimulus to the detector comprises a blower for directing the flow of stimulus from the testing device to the detector.

4. The system of claim 1 wherein the means for supplying the test stimulus to the detector comprises a tubing for directing the flow of stimulus from the testing device to the detector.

5. The system of claim 1 wherein the test medium is a liquid, gel, or solid.

6. The system of claim 1 wherein the test medium comprises a material which is transformed into the test stimulus when heated.

7. The system of claim 1 wherein the heating means is electrically operated.

8. The system of claim 3 wherein the blower is arranged to continue to blow air towards the direction of the detector after supplying the stimulus to the detector.

9. The system of claim 1 wherein the testing device further comprises a battery or rechargeable battery to provide power to the device.

10. The system of claim 1 further comprising a power supply means for providing mains power or low voltage power from another power source.

11. The system of claim 1 further comprising a control means for controlling the activation and deactivation of the testing device.

12. The system of claim 11 wherein the control means is in wired communication with the testing device.

13. The system of claim 11 wherein the control means is positioned remote from the testing device and is arranged to control the activation and deactivation of the testing device via wireless communication.

14. The system of claim 1 wherein the testing device comprises a heating chamber comprising on one side thereof a blocking member arranged to prevent stimulus generated by the testing device to be directed towards the detector when the blocking member is in the closed position.

15. The system of claim 14 wherein the heating chamber further comprises a closure element arranged to control the position of the blocking member.

16. A detection system for a gas and/or combustion product comprising:
a duct;
a detector for detecting the presence of the gas and/or combustion product in the channel; and
a testing device for testing the detector and comprising;
a source of test medium;
a heating means for heating the test medium in order to generate test stimulus representative of the gas and / or combustion product;
means for supplying the test medium to the heating means from the source; and
means for supplying the test stimulus to the detector,
wherein the testing device is positioned on the duct in a fixed position adjacent to the detector.

17. The system of claim 16 wherein both the detector and testing device are positioned in the elongate channel.

18. The system of claim 16 wherein the testing device is positioned in the elongate channel.

19. The system of claim 16 wherein the testing device is positioned on the duct outside the elongate channel and the means for supplying the generated stimulus to the detector comprises a tubing for directing the generating stimulus into the elongate channel.

20. The system of claim 16 wherein the testing device comprises a heating chamber comprising on one side thereof a blocking member arranged to prevent stimulus generated by the testing device to be directed towards the detector when the blocking member is in the closed position.

21. The system of claim 20 wherein the heating chamber further comprises a closure element arranged to control the position of the blocking member.

## Revendications

1. Système de détection pour un gaz et/ou un produit de combustion, comprenant :
un détecteur pour détecter la présence du gaz et/ou du produit de combustion, ce détecteur comprenant un boîtier ayant au moins une ouverture pour recevoir le gaz et/ou le produit de combustion à travers lui et fixé à une surface ;
un dispositif d'essai pour tester le détecteur, ce dispositif comprenant une source de milieux d'essai, un moyen de chauffage pour chauffer le milieu d'essai afin de générer un stimulus d'essai représentatif du gaz ou du produit de combustion, un moyen pour alimenter le milieu d'essai au moyen de chauffage à partir de la source, et un moyen pour alimenter le stimulus d'essai au détecteur, ce qui fait que, lorsqu'il est utilisé, le dispositif d'essai est agencé de façon à diriger le stimulus d'essai vers l'ouverture afin de tester le détecteur,
**caractérisé en ce que** le dispositif est monté sur la surface dans une position fixe adjacente au boîtier du détecteur et **en ce qu'**un nuage de gaz est émis vers le détecteur.

2. Système selon la revendication 1, dans lequel le détecteur et le dispositif d'essai forment une seule unité physique.

3. Système selon la revendication 1, dans lequel le moyen pour alimenter le stimulus d'essai au détecteur comprend une soufflante pour diriger l'écoulement de stimulus du dispositif d'essai au détecteur.

4. Système selon la revendication 1, dans lequel le moyen pour alimenter le stimulus d'essai au détecteur comprend une tubulure pour diriger l'écoulement de stimulus du dispositif d'essai au détecteur.

5. Système selon la revendication 1, dans lequel le milieu d'essai est un liquide, un gel ou un solide.

6. Système selon la revendication 1, dans lequel le milieu d'essai comprend une matière qui est transformée en le stimulus d'essai lorsqu'elle est chauffée.

7. Système selon la revendication 1, dans lequel le moyen de chauffage est électrique.

8. Système selon la revendication 3, dans lequel la soufflante est agencée de façon à continuer à souffler de l'air dans la direction du détecteur après l'alimentation du stimulus au détecteur.

9. Système selon la revendication 1, dans lequel le dispositif d'essai comprend en outre une batterie ou une batterie rechargeable pour fournir de l'énergie au dispositif.

10. Système selon la revendication 1 comprenant en outre un moyen d'alimentation électrique pour fournir une alimentation secteur ou une basse tension à partir d'une autre source d'énergie.

11. Système selon la revendication 1 comprenant en outre un moyen de commande pour commander l'activation et la désactivation du dispositif d'essai.

12. Système selon la revendication 11, dans lequel le moyen de commande est en communication câblée avec le dispositif d'essai.

13. Système selon la revendication 11, dans lequel le moyen de commande est positionné à distance du dispositif d'essai et est agencé de façon à commander l'activation et la désactivation du dispositif d'essai par communication sans fil.

14. Système selon la revendication 1, dans lequel le dispositif d'essai comprend une chambre de chauffage comprenant sur un côté de celle-ci un élément de blocage agencé de façon à empêcher le stimulus généré par le dispositif d'essai d'être dirigé vers le détecteur lorsque l'élément de blocage est dans la position fermée.

15. Système selon la revendication 14, dans lequel la chambre de chauffage comprend en outre un élément de fermeture agencé de façon à commander la position de l'élément de blocage.

16. Système de détection pour un gaz et/ou un produit de combustion, comprenant:
un conduit ;
un détecteur pour détecter la présence du gaz et/ou du produit de combustion dans le canal ; et
un dispositif d'essai pour tester le détecteur et comprenant :
une source de milieu d'essai ;
un moyen de chauffage pour chauffer le milieu d'essai afin de générer un stimulus d'essai représentatif du gaz et/ou du produit de combustion ;
un moyen pour alimenter le milieu d'essai au moyen de chauffage à partir de la source ; et
un moyen pour alimenter le stimulus d'essai au détecteur,
dans lequel le dispositif d'essai est positionné sur le conduit dans une position fixe adjacente au détecteur.

17. Système selon la revendication 16, dans lequel le détecteur et le dispositif d'essai sont tous deux positionnés dans le canal allongé.

18. Système selon la revendication 16, dans lequel le dispositif d'essai est positionné dans le canal allongé.

19. Système selon la revendication 16, dans lequel le dispositif d'essai est positionné sur le conduit à l'extérieur du canal allongé et le moyen pour alimenter le stimulus généré au détecteur comprend une tubulure pour diriger le stimulus généré dans le canal allongé.

20. Système selon la revendication 16, dans lequel le dispositif d'essai comprend une chambre de chauffage comprenant sur un côté de celle-ci un élément de blocage agencé de façon à empêcher le stimulus généré par le dispositif d'essai d'être dirigé vers le détecteur lorsque l'élément de blocage est dans la position fermée.

21. Système selon la revendication 20, dans lequel la chambre de chauffage comprend en outre un élément de fermeture agencé de façon à commander la position de l'élément de blocage.
